(19) Europäisches Patentamt
European Patent Office
Office européen des brevets

(11) Publication number: **0 230 013 B1**

# (12) EUROPEAN PATENT SPECIFICATION

(45) Date of publication of patent specification: **17.06.92**

(51) Int. Cl.5: **C07H 15/252**, A61K 31/70

(21) Application number: **86117662.6**

(22) Date of filing: **18.12.86**

## (54) New anthracycline derivatives, uses thereof as antitumor agent and production thereof.

(30) Priority: **18.12.85 JP 282798/85**

(43) Date of publication of application:
**29.07.87 Bulletin 87/31**

(45) Publication of the grant of the patent:
**17.06.92 Bulletin 92/25**

(84) Designated Contracting States:
**CH DE ES FR GB IT LI NL**

(56) References cited:

**THE JOURNAL OF ORGANIC CHEMISTRY, vol. 50, no. 24, 29th November 1985, pages 4913-4917, American Chemical Society; S. CASTILLON et al.: "Synthesis of 2'-C-fluoro-beta-daunomycin. An example of configurational retention in fluorodehydroxylation with diethylaminosulfur trifluoride"**

**THE JOURNAL OF ANTIBIOTICS, vol. XXXVII, no. 8, August 1984, pages 853-858, Japan Antibiotics Research Association, Tokyo, JP; D. HORTON et al.: "Synthesis and antitumor activity of 3'-deamino-3'-hydroxydoxorubicin. A facile procedure for the preparation of doxorubicin analogs"**

(73) Proprietor: **ZAIDAN HOJIN BISEIBUTSU KAGAKU KENKYU KAI**
**14-23, Kami Osaki 3-chome**
**Shinagawa-ku Tokyo(JP)**

(72) Inventor: **Umezawa, Hamao**
**23, Toyotama Kita 4-chome Nerima-ku**
**Tokyo(JP)**
Inventor: **Umezawa, Sumio**
**1, Banshu-cho Shinjuku-ku**
**Tokyo(JP)**
Inventor: **Tsuchiya, Tsutomu**
**1-17-201, Eda Higashi 3-chome Midori-ku**
**Yokohama-shi Kanagawa-ken(JP)**
Inventor: **Takeuchi, Tomio**
**1-11, Higashi Gotanda 5-chome Shingawa-ku**
**Tokyo(JP)**
Inventor: **Takagi, Yasushi**
**98-5, Higashi Kibohgaoka Asahi-ku**
**Yokohama-shi Kanagawa-ken(JP)**

(74) Representative: **Popp, Eugen, Dr. et al**
**MEISSNER, BOLTE & PARTNER Widenmayerstrasse 48 Postfach 86 06 24**
**W-8000 München 86(DE)**

**EP 0 230 013 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**THE JOURNAL OF ANTIBIOTICS, vol. XXXIX, no. 5, May 1986, pages 731-735, Japan Antibiotics Research Association, Tokyo, JP; K. MIURA: "Syntheses and antitumor activities of 7-O-(2,6-dideoxy-2-fluoro-alpha-L-talpyranosyl)-daunomycinone and -adriamycinone"**

## Description

SUMMARY OF THE INVENTION:

This invention relates to new anthracyclcine derivatives which are useful as antitumor agent. More particularly, this invention relates to 7-O-(2,6-dideoxy-2-fluoro-α-L-talopyranosyl) daunomycinone and 7-O-(2,6-dideoxy-2-fluoro-α-L-talopyranosyl) adriamycinone which are each a new compound useful as an antitumor agent. This invention also relates to a pharmaceutical, antitumor composition comprising the new anthracycline derivative as the active ingredient. This invention further relates to processes for the production of these new anthracycline derivatives.

BACKGROUND OF THE INVENTION:

Known examples of the antibiotics of anthracycline type include daunomycin (daunorubicin; U.S. Patent No. 3,6l6,242) and adriamycin (doxorubicin; U.S. Patent No. 3,590,028), which both may be obtained from the culture broth of a microorganism of actinomycetes. These two compounds exhibit a wide range of antitumor spectra against a variety of experimental tumors and have been used as a chemotherapeutic agent in the clinic practice. Daunomycin and adriamycin are the compound of the general formula

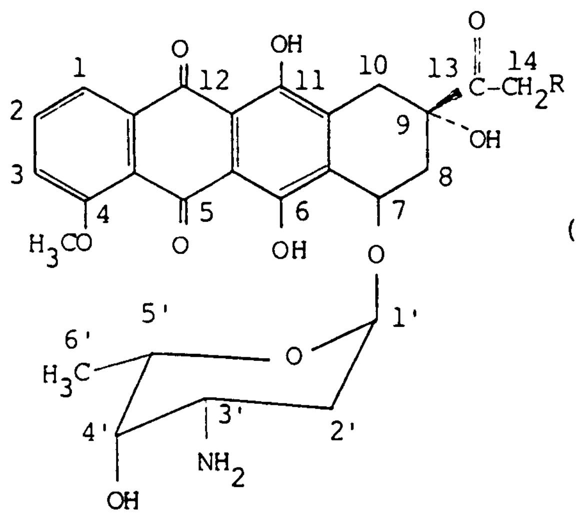

(a)

wherein R is a hydrogen atom or a hydroxyl group.

Daunomycin (the compound of the formula (a) above where R is the hydrogen atom) and adriamycin (the compound of the formula (a) where R is the hydroxyl group) can show a fairly high antitumor activity against various kinds of tumors. However, these two compounds are not necessarily antitumor agents which are completely satisfactory. Thus, daunomycin and adriamycin have been shown to exhibit wide antitumor spectra against the experimental tumors and also have widely been used as a valuable agent for the therapeutic treatment of tumors in the clinic practice. On the other hand, it is known that daunomycin and adriamycin can bring about heavy adverse side-effects that they can cause cardiac toxicity and a decrease in the number of leukocytes and falling-off of the hair in the patients who received the administration of these agents. It is reported that the glycoside linkage between the daunosaminyl group of the formula

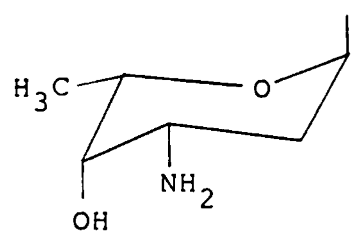

and the hydroxyl group at the 7-position of daunomycinone or adriamycinone is likely to be broken in vivo by the hydrolysis, and that the moiety of the aglycon as formed by the in vivo hydrolysis, namely the daunomycinone or adriamycinone shows a higher cardiac toxicity than the daunomycin or adriamycin itself.

In the past, therefore, some researches were already made in an attempt to provide new daunomycin-related compounds which possess a higher anticancer activity and a lower toxicity than daunomycin and adriamycin. For instance, studies for discovering and producing new daumonycin-analogous compounds by fermentative methods, semi-synthetic methods, total synthetic methods or enzymatical conversion methods were conducted. Such particular compounds previously proposed include, for example, aclacinomycins A and B (F. Arcamone "Topics in Antibiotic Chemistry" Vol. 2, pp. l02-279, published from Elis Horwood Limited, U.S. and U.S. Patent No. 3,988,3l5), 4′-O-tetrahydropyranyladriamycin (West Germany Patent No. 2,83l,579 and Japanese patent publication No. 47l94/8l) and N-mono-benzyl- or N-di-benzyl-adriamycin (U.S. Patent No. 4,l77,264).

Further, the specification of U.S. Patent No. 4,427,664 of Horton et al describes a chemical structure of compounds represented by the general formula

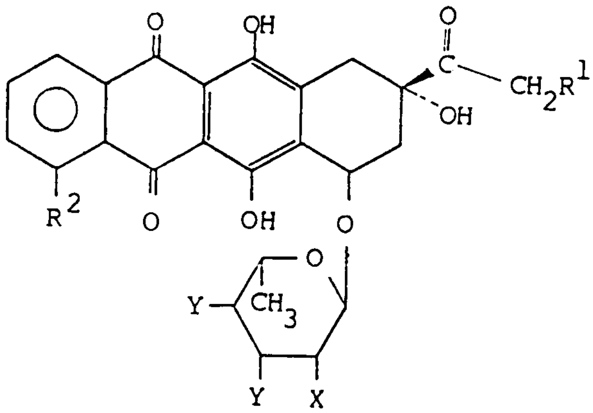

(b)

wherein $R^1$ is a hydrogen atom and $R^2$ is a methoxy group; or $R^1$ is a hydroxyl group and $R^2$ is a methoxy group; or $R^1$ and $R^2$ are each a hydrogen atom; or $R^1$ is a hydrogen atom and $R^2$ is a hydroxyl group, and X is an iodine, chlorine, bromine or fluorine atom and Y is a hydroxyl group or acetoxy group, and which compounds are of such structure that an aglycon selected from the group consisting of daunomycinone, desmethoxydaunomycinone, adriamycinone and carminomycinone is linked through at the oxygen atom at the 7-position thereof to the l′-position of a sugar of a 2′-halo-α-L-hexopyranose of the α-L-manno type or α-L-talo type. The method of producing a compound of the above formula (b) which is described in the specification of U.S. Patent No. 4,427,664 is the method wherein an aglycon such as daunomycinone and a glycal, for example, 3,4-di-O-acetyl-L-rhamnal or 3,4-di-O-acetyl-L-fucal, which corresponds to the sugar to be linked to said aglycon are dissolved together in substantially equimolar proportions in a mixture of aprotic organic solvents consisting of anhydrous acetonitrile and tetrahydrofuran and wherein to the resulting solution is then added an iodination agent such as N-iodosuccinimide together with a solvating agent such as dichloromethane at a low reaction temperature so that the aglycon reacts with the said glycal in such way that the glycal used is linked to the 7-hydroxyl group of the aglycon with accompanying by an alkoxyhalogenation of the glycal. According to this method of Horton et al, it happens as described in the specification of said U.S. Patent No. 4,427,664, that the halogen atom as possessed by the halogenation agent employed is introduced into the 2′-position of the sugar moiety of the compound of the formula (b) formed as the reaction product, and that for instance, when N-iodosuccinimide is employed as the iodination agent, the iodine atom is introduced into the 2′-position of the sugar moiety of the reaction product as obtained.

The U.S. Patent No. 4,427,664 specification discloses an Experimental Example n which 3,4-di-O-acetyl-L-rhamnal of the formula

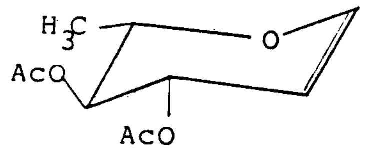

(c)

where Ac denotes an acetyl group here and also hereinafter unless otherwise stated is reacted with daunomycinone and N-iodosuccinimide, with accompanying alkoxyhalogenation of said rhamnal compound, to produce 7-O-(3,4-di-O-acetyl-2,6-dideoxy-2-iodo-α-L-manno-hexopyranosyl) daunomycinone, as well as another Experimental Example in which 3,4-di-O-acetyl-L-fucal of the formula

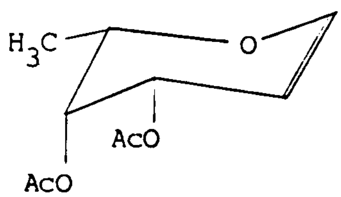

(d)

is reacted with daunomycinone and N-iodosuccinimide with accompanying by the alkoxyhalogenation of said fucal compound, to produce 7-O-(3,4-di-O-acetyl-2,6-dideoxy-2-iodo-α-L-talo-hexopyranosyl) daunomycinone. However, this U.S. Patent specification does not disclose any further experimental Examples.

In the U.S. Patent No. 4,427,664 specification, the formula (b) apprearing therein refers to that X may broadly be an iodine, bromine, chlorine or fluorine atom, but there is not shown any Experimental Examples in which such a compound of the formula (b) where X is the bromine, chlorine or fluorine atom was virtually synthetized. If the one skilled in the art wishes to synthetize a compound of the above formula (b) where X is the bromine, chlorine or fluorine atom, it is expected that in accordance with the method of producing the compound of the formula (b) taught in the U.S. Patent No. 4,427,664 specification, he will repeat the procedures of the two Experimental Examples as given in said U.S. patent specification using N-bromosuccinimide, N-chlorosuccinimide or N-fluorosuccinimide as the halogenation agent in place of the N-iodosuccinimide employed by Horton et al. Among the above-mentioned three compounds which are expectedly employable as the halogenation agent in place of the N-iodosuccinimide, a substance which is to be represented by the formula

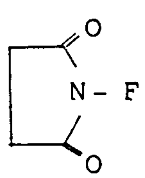

(e)

and which may be termed as N-fluorosuccinimide is unknown by now, because it is not described before in any literatures with respect of any process of preparing it and with respect of the physical and chemical properties of it, as far as we, the present inventors, have searched numerous literatures. Accordingly, it is evidently considered that a compound which may be termed as N-fluorosuccinimide is an actually unknown substance which was never prepared in the past prior to these days.

Besides, it is to be noted that the fluorine element which may be and have usually been considered to belong to the class or family of halogens is possessing an extraordinarily different and higher electronegativity than the other halogen elements, iodine, bromine and chlorine, and also that, as be well known, the fluorine element shows the chemical behaviors very much different from those of the other halogen elements. Accordingly, even when it is assumed that the N-fluorosuccinimide will have been prepared by any chemical process, it is highly probable that the properties of the chemical linkage between the fluorine atom and the succinimide group existing in the N-fluorosuccinimide as an imaginable compound would be very much different from the properties of the other sorts of a halo group linking to the succinimide group, and that such fluoro group is too strongly or too weakly linking to the succinimide group. For these reasons,

it is very much hardly conceivable that the N-fluorosuccinimide can act as a fluorination agent to transfer its fluoro group into a second compound and bring about the fluorination of the latter compound. Accordingly, it is not presumable that the N-fluorosuccinimide, even if prepared, can serve especially as the fluorination agent required in the method of producing the compounds of the formula (b) which is described in the U.S. Patent No. 4,427,664 specification.

In short, the U.S. Patent No. 4,427,664 specification describes the chemical structure of the compounds of the above formula (b) where X may broadly be a chlorine, bromine, iodine or fluorine atom. Of the compounds which are designated by the above formula (b), the compounds of the formula (b) where X is a chlorine or bromine atom are shown merely with reference to their chemical structure in the U.S. Patent No. 4,427,664 specification but were actually not synthetized concretely. Nonetheless, it is admittable that N-chlorosuccinimide and N-bromosuccinimide are a chlorination or bromination agent already known and be necessary and available as the halogenation agent in the process of producing such compound of the formula (b) where X = chlorine or bromine, according to said U.S. patent of Horton et al, and therefore it is deducible from the descriptions of the U.S. Patent No. 4,427,664 specification that the production of such compounds of the formula (b) where X = chlorine or bromine and isolation of such compounds as produced are possible theoretically in accordance with the method of Horton et al as disclosed in said U.S. patent specification. In contrast, however, it is evident that the U.S. Patent No. 4,427,664 specification does not disclose or teach a process for really producing such compound of the formula (b) where X is the fluorine atom, to such extent that the process would be workable by chemical experts in view of the disclosure of the U.S. Patent No. 4,427,664, firstly, because the N-fluorosuccinimide which is deemed as necessary as the fluorination agent for the production of the compound of the formula (b) where X = fluorine, according to the disclosed method of Horton et al is a substance which is still unknown up to these days and is very much hardly predictable to be able to act as the necessary fluorination agent for the intended purpose, and secondly, because the U.S. Patent No. 4,427,664 of Horton et al does nowhere teach how to prepare the N-fluorosuccinimide. Hence, it is worthy to say that such compound of the above formula (b) where X is the fluorine as shown in the U.S. Patent No. 4,427,664 was a merely imaginary one which was thought by referring to its chemical structure on the papers in the specification of said U.S. patent and of which utility for the intended fluorination agent is very much suspicious. Accordingly, we, do not believe that such special compound of the formula (b) where X is the fluorine atom could be prepared by the chemical experts in view of the disclosure of the U.S. Patent No. 4,427,664.

Furthermore, in the U.S. Patent No. 4,427,664 specification, there is described by the inventors, Horton et al that the compounds of the formula (b) exhibit the antitumor activities against mouse blood cancer, Leukemia P 388. More particularly, this U.S. patent specification describes such antitumor activity of 7-O-(3,4-di-O-acetyl-2,6-dideoxy-2-iodo-α-L-mannohexopyranosyl) daunomycinone (nominated as Compound NSC 331,962 by Horton et al) as tested against Leukemia P 388 but does not describe any data of the antitumor activity of 7-O-(3,4-di-O-acetyl-2,6-dideoxy-2-iodo-α-L-talohexopyranosyl)daunomycinone (nominated as Compound NSC 327,472 by Horton et al). While, according to an article of Horton et at reported in the "Carbohydrate Research" Vol. l36, pp. 39l-396 (l985), they obtained experimental results to show that said Compound NSC 33l,962 exhibited an anitumor activity that the increase (in %) of survival days of the mice treated, compared to the mice untreated (Control) (namely, T/C, %) was 247% at a dosage of 50 mg/kg of the test compound when the mice as inoculated with Leukemia P 388 were treated by administration of the test compound; and that said Compound NSC 33l,962 exhibited an antitumor activity that the increase (in %) of survival days of the mice treated, as compared to the mice untreated (T/C, %) was l96% at a dosage of 25 mg/kg of the test compound when the mice as inoculated with Leukemia L-l2l0 were treated by administration of the test compound (a single dose per day, intraperitoneally given for 9 days). Also, the above-mentioned Compound NSC 327,472 experimentally showed such antitumor activities that the increase (%) of survival days of the Leukemia P 388-inoculated mice treated was l72% at a dosage of l2.5 mg/kg to 25 mg/kg of Compound NSC 327,472, whereas the increase (%) of survival days of the Leukemia P 388-inoculated mice treated decreased to l62% at a further increased dosage of l50 mg/kg of the tested compound.

Apart from the above-mentioned researches of Horton et al, we have made studies in an attempt to produce new daunomycin derivatives or adriamycin derivatives which have better antitumor activity and lower toxicity than daunomycin and adriamycin. As a result, we already succeeded to synthetize a few examples of such daunomycin derivative and adriamycin derivative in which the sugar moiety of daunomycin or adriamycin has chemically been modified and which are useful as the antitumor agent. Thus, we reported 4′-O-tetrahydropyranyl-daunomycins and -adriamycins (Japanese patent publication No. 47l94/8l); and 3′-deamino-3′-morpholino-daunomycins and -adriamycins (Japanese patent application first publication "KOKAI" No. l63393/82).

Further, we have made another studies to provide new compounds which are derived by chemical modification with a fluoro group of the 3′-position or 2′-position of kanamycin A and kanamycin B of the aminoglycosidic antibiotics. Thus, we have succeeded to synthetize 3′-deoxy-3′-fluorokanamycin A (Japanese patent application No. 161615/84; U.S. patent application SN. 758,819; European patent application No. 85 401575.7); 3′-deoxy-3′-fluorokanamycin B (Japanese patent application No. 262700/84); and 2′, 3′-dideoxy-2′-fluorokanamycin A (Japanese patent application No. 263759/84; U.S. Patent application SN. 807,485; European patent application No. 85 115901.2).

In this way, we already obtained many findings and experiences in the fluorine chemistry of sugars through our studies where the fluoro group is introduced into kanamycins of the glycosidic antibiotics. Based on these findings and experiences, we have now succeeded to synthetize as a new compound a 4-O-benzyl-protected derivative of methyl 2,6-dideoxy-2-fluoro-α-L-idopyranoside represented by the formula

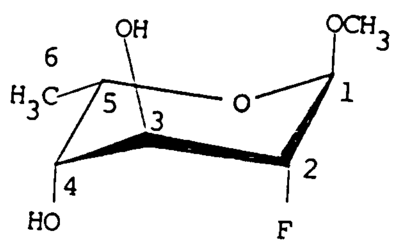
(g)

through a multi-stage process with starting from L-fucose of the formula

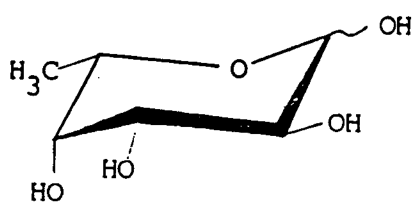
(f)

Further, we succeeded to synthetize from the sugar compound of the above formula (g) methyl 2,6-dideoxy-2-fluoro-α-L-talopyranoside of the formula

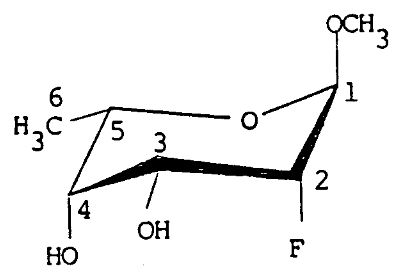
(h)

as a new compound, and also synthetize from the compound of the above formula (h) a 3,4-di-O-protected-2,6-dideoxy-2-fluoro-α-L-talopyranosyl halide of the formula

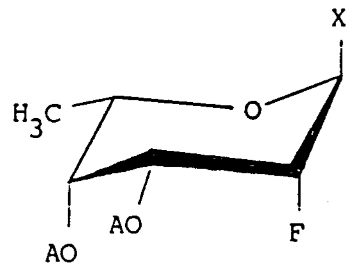

(i)

wherein A is a hydroxyl-protecting group, particularly an acyl group, especially a lower alkanoyl group such as acetyl or an aroyl group such as benzoyl and X is a chlorine, bromine or iodine atom, for example, 3,4-di-O-acetyl-2,6-dideoxy-2-fluoro-α-L-talopyranosyl bromide as a new compound.

Then, we have now succeeded to produce firstly 7-O-(2,6-dideoxy-2-fluoro-α-L-talopyranosyl) daunomycinone of the formula

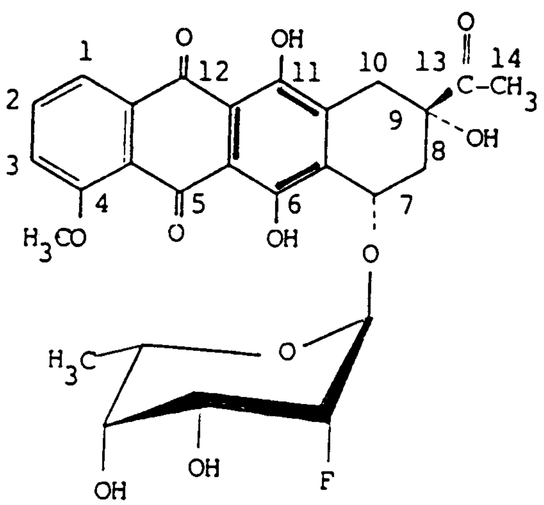

(j)

as a new compound (this compound is referred to by the formula (Ib) hereinafter), by reacting the 3,4-di-O-protected-2,6-dideoxy-2-fluoro-α-L-talopyranosyl halide of the above formula (i) with the 7-hydroxyl group of daunomycinone and then removing the residual hydroxyl-protecting groups (A) from the resulting reaction product.

Furthermore, by converting the 14-methyl group of the compound of the above formula (j) into a hydroxy-methyl group ($-CH_2OH$) by treatment with a mild oxidizing agent, we have now succeeded to produce firstly 7-O-(2,6-dideoxy-2-fluoro-α-L-talopyranosyl)adriamycinone of the formula

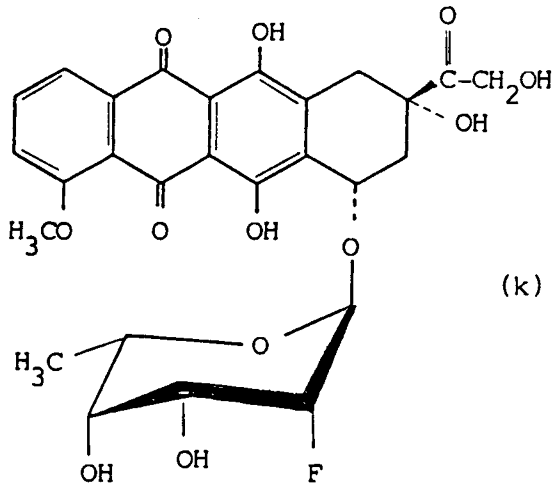

(k)

as a new compound (this compound is referred to by the formula (Id) hereinafter). We also have found that the new compound of the formula (j) and the new compound of the formula (k) have excellent antitumor activities and low toxicities and that the glycoside linkage at the 7-hydroxyl group of these new compounds shows a high stability against hydrolysis by acid. Accordingly, the new compound of the formula (j) and the compound of the formula (k) are interesting for use as antitumor agent owing to their low toxicities coupled with their excellent antitumor activities as demonstrated hereinafter. These new compounds of the formulae (j) and (k) have also high antibacterial activities and are useful as antibacterial agent.

DETAILED DESCRIPTION OF INVENTION:

According to a first aspect of this invention, therefore, there is provided an anthracycline derivative represented by the general formula

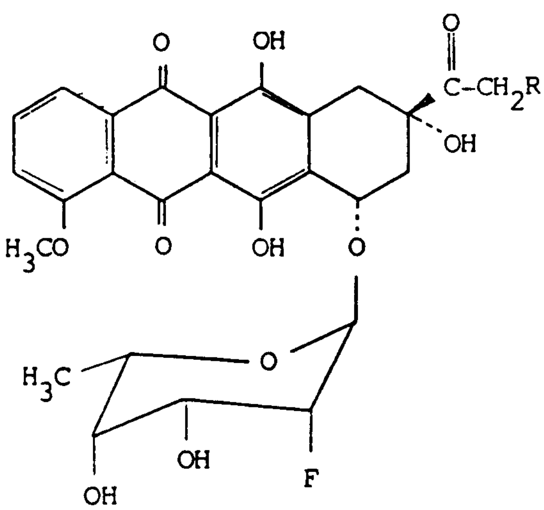

(I)

wherein R is a hydrogen atom or a hydroxyl group.

Of the compounds of the formula (I) according to this invention, 7-O-(2,6-dideoxy-2-fluoro-α-L-talopyranosyl)daunomycinone of the formula (j) (referred to as Compound No. I of this invention sometimes hereinafter) is in the form of red colored solid having a specific optical rotation $[\alpha]_D^{25}$ + 197° (c 0.02, chloroform-methanol (1:1)). 7-O-(2,6-dideoxy-2-fluoro-α-L-talopyranosyl)adriamycinone of the formula (k) (referred to as Compound No. 2 of this invention sometimes hereinafter) is in the form of red colored solid having a specific optical rotation $[\alpha]_D^{25}$ + 194° (c 0.01, chloroform-methanol (1:1)).

We have confirmed by animal tests that the compounds of formula (I) according to this invention exhibit significantly high antitumor activities on experimental tumors and that the level of said activities is much

higher than those of daunomycin and adriamycin with an acceptably low level of toxicities. Some typical tests on experimental animal tumors are given below.

Test I

Antitumor activity on leukemia in $CDF_1$ mice caused by Leukemia L-l2l0 cells

In this test, Compound Nos. I and 2 of this invention were assessed in comparison with daunomycin and adriamycin on their tumor inhibitory activity against leukemia in mice. Thus, $CDF_1$ mice were intraperitoneally transplanted with cells of Leukemia L-l2l0 ($l \times l0^5$ cells/mouse). From 24 hours after i.p. transplantation of L l2l0 cells, each compound to be tested was administered intraperitoneally for 9 consecutive days at once per day. Observation was made for 60 days where survival days of each mouse tested were recorded and percentage increase in lifespan of the treated mice, in comparison with control test wherein mice were treated only, with the administration of physiological saline, was calculated as T/C % where T is the mean survival days of the treated animals and C is the mean survival days of the untreated control animals.The test results are shown in Table I.

Table 1

| Compound tested | % Increase in life-span (T/C) Dose mg/kg/day 5 | 2.5 | 1.25 | 0.6 | 0.3 | 0.15 |
|---|---|---|---|---|---|---|
| 7-O-(2,6-Dideoxy-2-fluoro-α-L-talopyranosyl)daunomycinone (Compound No. 1 of this invention) | 184 | 217 | 171 | 125 | 105 | 105 |
| Daunomycin (comparison) | 138* | 171* | 158 | 145 | 112 | 132 |
| 7-O-(2,6-Dideoxy-2-fluoro-α-L-talopyranosyl)adriamycinone (Compound No. 2 of this invention) | >740 | >352 | 275 | 185 | 182 | 127 |
| Adriamycin (comparison) | 191* | 228 | 222 | 142 | 136 | 123 |

* shows that a toxicity or its sign of the compound tested in the animals tested such as death due to toxicity or weight loss was observed.

Adriamycin used in Test I above for comparison purpose is an anticancer agent clinically used in dosage of 0.4 - 2 mg/kg depending upon the nature of cancer to be treated. When administered to mice inoculated with L-l2l0 cells in dosage of 2.5 mg - 5 mg/kg/day, adriamycin exhibits an antitumor effect to a certain extent, i.e. percentage increase in life-span of 228 - l9l% with appearance of toxicity (see Table I). In contrast therewith, Compound No. I [7-O-(2,6-dideoxy-2-fluoro-α-L-talopyranosyl)daunomycinone] of this invention in the same dosage as above (i.e. 2.5 - 5 mg/kg/day) shows a percentage increase in life-span of

2l7 - l84% with no sign of toxicity, and Compound No. 2 [7-O-(2,6-dideoxy-2-fluoro-α-L-talopyranosyl)-adriamycinone] of this invention in the same dosage as above shows a significantly high percentage increase in life-span of >352% - >740% with no sign of toxicity. Such superior effects exhibited by the compounds of this invention to that of adriamycin are to be noticeable. Based on this, the new anthracycline derivatives of this invention are believed to have better antitumor activities than those of adriamycin and are expected to be very useful as antitumor agent for clinical applications, including a variety of tumor diseases in human being to which adriamycin has been applied. On the other hand, Compound NSC 33l,962 synthesized by Horton et al. as referred to above exhibits only a level of antitumor activity of percentage increase in life-span of l96% even when administered in such a high dosage as 25 mg/kg/day to mice inoculated with L-l2l0 cells. This level of antitumor activity is apparently lower than that of adriamycin.

A further advantage of the anthracycline derivatives of formula (I) according to this invention is in that the susceptibility of the glycoside linkage to be broken by acid hydrolysis is very low. This is, we believe, to be one of reasons why the compounds of this invention are of low toxicity. Thus, our experiment has shown that Compound No. l of this invention, when subjected to acid hydrolysis with l N HCl in a mixture of acetonitrile-water (4:l) at 60°C for 8 hours, remains substantially as it is,without being decomposed, whereas daunomycin, when subjected to acid hydrolysis with 0.2 N HCl in the same mixture of acetonitrile-water at 60°C, is completely decomposed in only 30 minutes due to breakage of the glycoside linkage, resulting in losing its anticancer activity. Of course, a high chemical stability of a compound in vitro is not always related to its stability against hydrolysis in vivo, but it may be assumed in view of high anticancer effects in vivo of the compounds of this invention that the glycoside linkage of those compounds is of high stability in vivo, too. In any case, it is true that such high chemical stability of the glycoside linkage of the compounds of this invnetion is advantageous in that it makes the handling of those compounds easy in chemical applications. Test 2 given below illustrates the high stability of the glycoside linkage of the compounds of this invention.

Test 2

(a) Acid hydrolysis of daunomycin

Daunomycin (l mg) was dissolved in a mixture (0.l ml) of 0.2 N*HCl-80% $CH_3CN$-$H_2O$ (* the 0.2 N concentration of HCl being of the said mixture) and the solution was heated in an oil bath at 6l - 62°C for 30 minutes to cause the hydrolysis. Analysis of the reaction solution by silica gel thin layer chromatography (TLC) showed disappearance of the spot of daunomycin, but appearance of both the spot of daunomycinone and a spot possibly of the sugar moiety which appears at Rf = 0 when eluted with benzene-acetone (l:l by volume) as eluent and which was colored in black by spraying with sulfuric acid followed by heating. The reaction solution was allowed to stand at room temperature to deposit daunomycinone as red crystals.

(b) Acid hydrolysis of Compound No. l (abbreviated as FTDM) of this invention

FTDM (0.7 mg) was suspended in a mixture (0.l ml) of 0.2 N HCl-80% $CH_3CN$-$H_2O$ and the suspension was stirred in an oil bath at 6l - 62°C for 30 minutes. Analysis of the reaction mixture by TLC showed the presence of the spot of FTDM only.

A futher amount (0.3 ml) of the mixture of 0.2N HCl-80% $CH_3CN$-$H_2O$ was added to the reaction mixture, when the mixture substantially became a solution with a little amount of insolubles. Then, it was heated to 6l-62°C under stirring to give a homogeneous solution. The solution was then heated at that temperature for further 3 hours and analyzed by TLC with the result that there appeared the spot of FTDM only.

An amount (0.23 ml) of a mixture of 2.4N HCl-80% $CH_3CN$-$H_2O$ was then added to the resulting solution, where the overall concentration of HCl became l N HCl, and the mixture was further heated in an oil bath at 6l-62°C for 8 hours and then analyzed by TLC with the result that a spot of a trace amount of daunomycinone appeared, but most of FTDM was also present as it was.

In our opinion, the high stability against hydrolysis of the glycoside linkage of the anthracycline compounds of this invention is attributable to the presence of fluoro group attached on the 2′-position of the sugar moiety of the compounds. It is well-known in the art that amongst elements of halogens, fluorine is a unique element as compared with other halogens and thus cannot be dealt with in chemical behavior on the same level as other halogens. It is also known from the point of view of physical chemistry that the electro-negativity (X) of fluorine is 4.0 and those of chlorine, bromine and iodine are 3.0, 2.8 and 2.5, respectively,

and the bond energy of C-F bond is ll6 Kcal/mole and those of C-Cl, C-Br and C-I bonds are 77, 64 and 5l Kcal/mole, respectively. The 2′-fluoro group on the sugar moiety of the compound of this invention strongly attracts electrons into the fluorine atom through the C-F bond due to such a high electro-negativity of fluorine, as a result of which the electron density of the two oxygen atoms attached to the l′-carbon atom of the sugar moiety is lowered and thus these two oxygen atoms show a tendency not to attract any proton $H^+$ from outside, so that the glycoside linkage adjacent to the l′-carbon atom becomes difficult to be broken by hydrolysis, and in other words, the stability of the glycoside linkage against hydrolysis has been enhanced. Further, the 2′-fluoro group and the oxygen atom of the glycoside linkage attached to the l′-carbon atom are in antiperiplanar relation, so that the former has a very high power for attracting electrons from the latter. Accordingly, the 2′-fluoro group on the sugar moiety of the anthracycline compounds of this invention can have a significant effect for enhancing the stability against acid hydrolysis of the glycoside linkage adjacent to the l′-carbon atom. Such glycoside linkage-stabilizing effect of the 2′-fluoro group is thus much higher than that of the 2′-iodo group in the above-mentioned Compound NSC 33l,962 and Compound NSC 327,472 both synthesized by Horton et al.

Further, according to our thought, the reason why the compounds of formula (I) characterized by the 2′-fluoro group on the sugar moiety can exhibit antitumor activities much higher than those exhibited by the above-said Compound NSC 33l,962 and Compound NSC 327,472 of Horton et al. may be explained as follows:

The fluorine atom attached to the 2′-carbon atom of the compounds of this invention has a Van der Waals' radius (i.e. a value indicating the bulkiness of an atom) of l.35. This value is next to that of hydrogen atom being l.20 which is the smallest radius and is far smaller than those of chlorine, bromine and iodine atoms being l.8l, l.95 and 2.l5, respectively. According to the Arcamone's opinion given in his paper "Doxorubicin" (see "Medicinal Chemistry",Ser. l7 (l98l) published by Academic Press, New York), it is suggested that the presence of a substituent at the 2′-position on the sugar moiety of daunomycins makes their antitumor activities lower or null. It is quite surprising from Arcamone's suggestion above that the compounds of formula (I) according to this invention exhibit significantly high antitumor activities in spite of the presence of the 2′-substituent. One possible explanation for this is such that the bulkiness of the fluorine atom as the 2′-substituent in the compounds of this invention is very low, i.e. l.35 expressed as Van der Waals' radius which is next to the lowest value l.20 for hydrogen atom, so that the presence of the 2′-fluoro substituent of such low bulkiness gives little or a little influence only on the space and steric configurations of the molecule of the compounds, that is no steric hindrance occurs, thus resulting in no deactivation of the desired antitumor activities of the compounds. In view of this, it may also be explained that anthracycline compounds having similar structure to that of the compounds of formula (I) according to this invention, but having the 2′-chloro, 2′-bromo or 2′-iodo substituent in place of the 2′-fluoro substituent have appreciably lower antitumor activities than those of the compounds of this invention.

As is clear from the test results given above, the compounds of formula (I) according to this invention exhibit excellent antitumor activities to Leukemia L-l2l0 cells and experimental animal tumors.

Therefore, the compounds of formula (I) acccording to this invention can be used as antitumor agents, perticularly for treatment of malignant tumors, including solid tumors and ascites tumors.

According to the second aspect of this invention, therefore, there is provided an pharmaceutical antitumor composition comprising an anthracycline derivative represented by the general formula

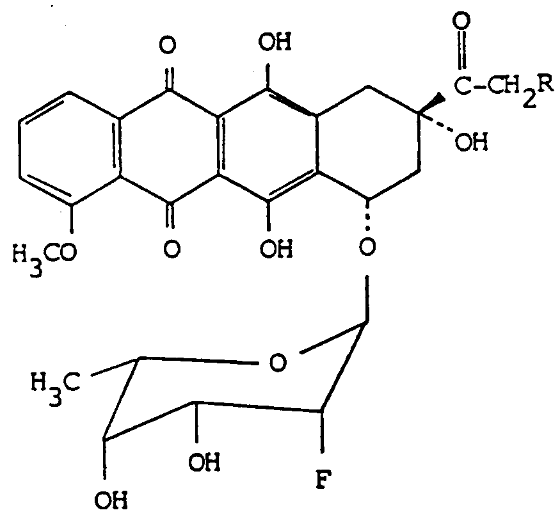

(I)

where R is a hydrogen atom or a hydroxyl group as the active ingredient, in association with a pharmaceutically acceptable carrier for the active ingredient.

When the new compound of this invention is administered to patients in practice, it may normally be given through non-oral routes. While, the new compound of this invention may be formulated into known preparations such as tablet and syrup for oral administration by mixing with conventional excipients as known in the pharmaceutical field so that it is given orally, because the compound of this invention is highly stable against the acidic hydrolysis in vivo.

For the administration of the new compound of this invention to animals, the compound may be formulated into an injectable solution or suspension suitable for intraperitoneal injection, subcutaneous injection, blood vessel injection, either intravenous or intra-arterial, and local injection. For the administration to human, the new compound of this invention may be formulated into injectable preparations suitable for blood vessel injection, either intravenous or intra-arterial, and local injection.

The dosage of the new compound of this invention may be decided in account of the results of animal tests and various factors, and the compound of this invention may be administered in dosage units continuously or intermitently so that the total dosages do not exceed a given value. Of course, however, the amount of the compound of this invention as administered may be changed appropriately with taking into account the administration route; conditions of patients or animals to be treated, such as age, body weight, sex, sensitivity, foods, time, duration, and way of administration; sort of the medicinal agents given concurrently; and symptons of diseases. For a guideline, a normal dosage of the new compound of this invention may be as high as the known dosage of adriamycin upon use as the antitumor agent and may be in a range of 0.4 mg/kg to 2 mg/kg per unit dose a day. Optimum dosage and optimum numbers of administration of the compound of this invention are necessary to be decided by the medicinal experts through preliminary tests for determination of optimum dosage in view of the above-mentioned guideline. The above requirements for administration are applied to upon the oral administration of the new compound of this invention.

Moreover, the new compound of the formula (I) according to this invention shows antibacterial activity against gram-positive bacteria and may be administered as an agent for therapeutic treatment of diseases caused by gram-positive bacteria infection. When the compound of this invention is used as antibacterial agent, the formulation and dosage of the active compound may be the same as mentioned above for the antitumor agent. Suitable dosage and numbers of the administration as well as the forms of the preparation may be decided by the skilled in the art with taking the pre-cautions same as mentioned above.

Now, the production of the compound of the formula (I) according to this invention is described.

Of the compounds of the formula (I), such compound of the formula (I) where R is the hydrogen atom, specifically the 7-O-(2,6-dideoxy-2-fluoro-α-L-talopyranosyl) daunomycinone of the above formula (j) or of the formula (Ib) given hereinafter (namely, Compound No. I of this invention) may be synthetized by reacting a 3,4-di-O-protected-2,6-dideoxy-2-fluoro-α-L-talopyranosyl halide of the formula (i) given hereinbefore, that is, the compound of the formula (III) given below, with the 7-hydroxyl group of daunomycinone, and then removing the residual hydroxyl-protecting groups from the reaction product obtained, where such residual hydroxyl-protecting groups are remaining in the reaction product.

According to the third aspect of this invention, therefore, there is provided a process for the production

of an anthracycline derivative represented by the formula

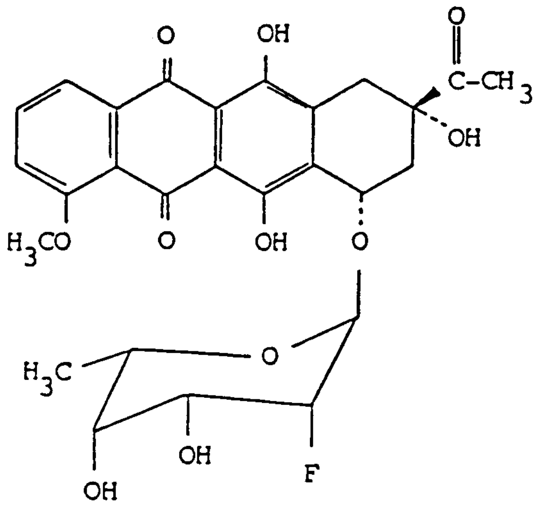

(Ib )

which comprises reacting daunomycinone of the formula

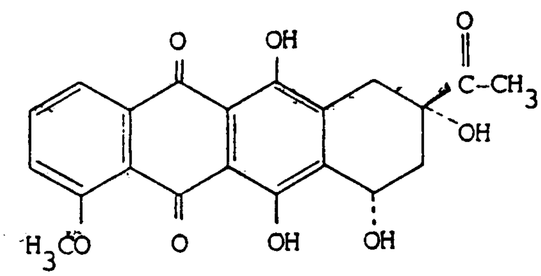

(II)

with a 2,6-dideoxy-2-fluoro-α-L-talopyranosyl halide of the formula

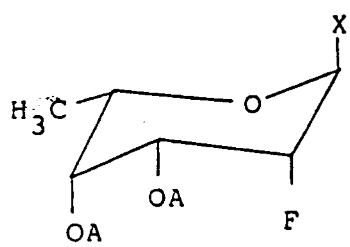

(III)

wherein X is a bromine, iodine or chlorine atom and A is a hydroxyl-protecting group or a hydrogen atom, to produce an anthracycline derivative of the formula

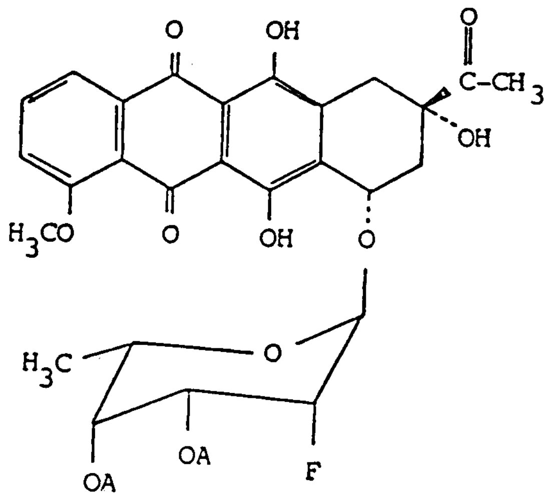

(Ia)

wherein A is as defined above, and then removing the hydroxyl-protecting groups (A) from the compound of the formula (Ia) in a known manner, when the compound of the formula (Ia) is containing the hydroxyl-protecting groups (A) remaining therein.

In the process according to the third aspect of this invention, the reaction between daunomycinone of formula (II) and a 2,6-dideoxy-2-fluoro-α-L-talopyranosyl halide or its 3,4-di-O-protected derivative of formula (III) may be carried out by a known process for condensing an aglycone with a sugar through a glycoside linkage.

According to this process, in general, the reaction between daunomycinone of formula (II) and a compound of formula (III) may usually be effected in an aprotic organic solvent such as N,N-dimethylformamide (hereinafter referred to as DMF), dimethylsulfoxide, hexamethylphosphorictriamide, diglyme, tetrahydrofuran, dioxane and various halogenated hydrocarbons such as dichloromethane, chloroform, dichloroethane, trichloroethane and tetrachloroethane. Desirably, such aprotic solvent is dried to be free from water before use although the presence of a little amount of water therein is acceptable.

Usually, the said condensation reaction is desirably carried out in the presence of a dehydrohalogenation agent, for example, tertiary amines such as tertiary alkylamines e.g. triethylamine, and dimethylaniline; trimethylsilyl triflate, silver oxide, silver trifluoromethanesulfonate, silver carbonate, mercuric oxide, mercury bromide and mercury cyanide.

The amount of such dehydrohalogenation agent to be used may generally be at least l mole, preferably 2.5-4.0 moles per mole of the compound of formula (III).

The amount of the compound of formula (III) to be used is desirably in a slight excess to the stoichiometric amount, for example, l.5 moles, per mole of the compound of formula (II).

The reaction temperature is not so limitative, but may generally be in the range from the freezing point of the solvent used to 80 °C and conveniently at or around the room temperature. Preferably, the reaction between the compound of formula (II) and a compound of formula (III) is carried out in an aprotic organic solvent, typically a halogenated hydrocarbon such as dichloromethane, chloroform, dichloroethane, trichloroethane and tetrachloroethane, under anhydrous conditions, in the presence of a condensation catalyst such as mercuric oxide and mercuric bromide and preferably in the co-presence of a molecular sieve as dehydrating agent. In this preferred embodiment of the process, the reaction may be effected by using daunomycin one (II) and a compound of formula (III) in equimolar proportion or in a slight excess proportion of the latter to the former. The reaction temperature may be in the range of -20 °C to 50 °C The reaction product of formula (Ia) may be recovered from the reaction solution in a usual manner. The reaction product of formula (Ia) thus recovered may be purified by column chromatography on silica gel using a mixture of benzene and acetone as eluent.

When the compound of formula (Ia) contains the hydroxyl-protecting groups remaining therein, the protecting groups may be removed by any known deprotecting method. The hydroxyl-protecting groups (A) each are usually an acyl group which may be removed by hydrolysis in the presence of an alkali metal hydroxide such as sodium hydroxide and water.

The resulting compound of formula (Ib), 7-O-(2,6-dideoxy-2-fluoro-α-L-talopyranosyl)daunomycinone, is in the form of a red solid and may be purified by reprecipitation or recrystallization from a mixture of organic solvents such as chloroform-hexane.

A compound of formula (I) where R is hydroxyl, or the compound of formula (Id), namely 7-O-(2,6-dideoxy-2-fluoro-α-L-talopyranosyl)adriamycinone of formula (k) above, may be synthesized by converting the l4-methyl group of either the compound of formula (la) obtained as an intermediate by the process according to the third aspect of this invention or the compound of formula (lb) obtained in said process as final product into a hydroxymethyl ($-CH_2OH$) group and, if the product so converted contains the hydroxyl-protecting groups remaining therein, by removing the said hydroxy-protecting groups from the product.

According to the fourth aspect of this invention, therefore, there is provided a process for the production of an anthracycline derivative of the formula

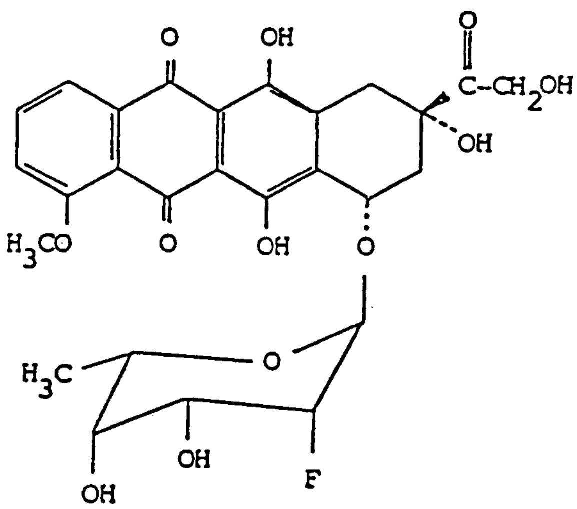

(Id)

which comprises reacting daunomycinone of the formula

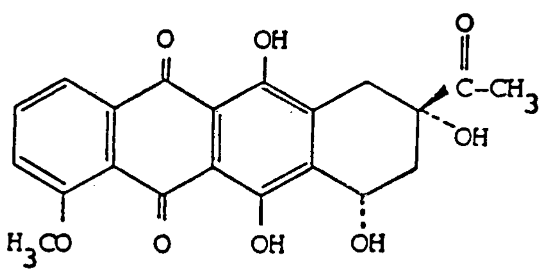

(II)

with a 2,6-dideoxy-2-fluoro-α-L-talopyranosyl halide of the formula

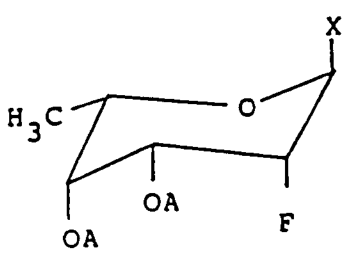

(III)

wherein X is a bromine, iodine or chlorine atom and A is a hydroxyl-protecting group or a hydrogen atom, to produce an anthracycline derivative of the formula

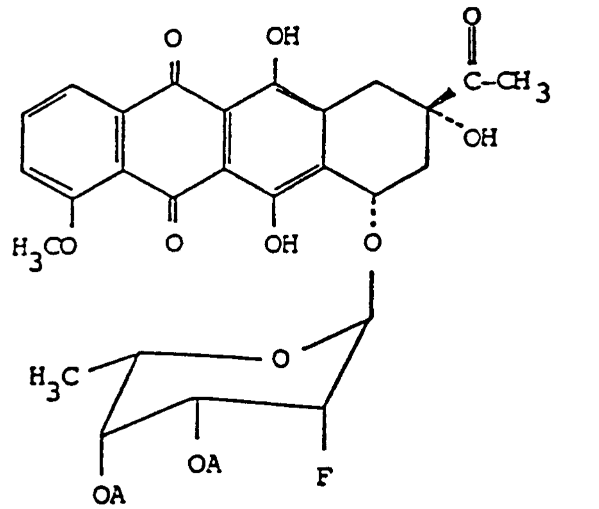

(Ia)

wherein A is as defined above, and then, either, removing the hydroxyl-protecting groups (A) from the compound of the formula (Ia) when the compound of the formula (Ia) is containing the hydroxyl-protecting groups (A) remaining therein, to produce the deprotected product having deprived of the hydroxyl-protecting groups (A) and subsequently converting the methyl group at the l4-position of said deprotected product into a hydroxymethyl group to produce the compound of the formula (Id) above, or converting the methyl group at the l4-position of said compound of the formula (Ia) into the hydroxymethyl group to give an anthracycline derivative of the formula

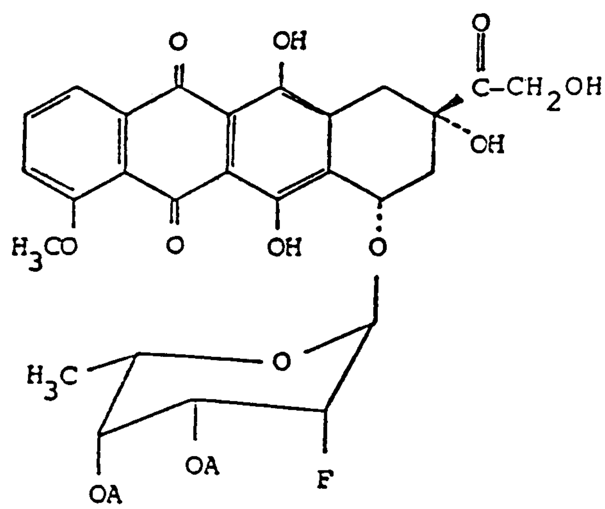

(Ic)

wherein A is as defined above and subsequently removing the hydroxyl-protecting groups (A) from the compound of the formula (Ic) when this compound of the formula (Ic) is containing the hydroxyl-protecting groups (A) remaining therein, to produce the compound of the formula (Id) above.

In the process according to the fourth aspect of this invention, the reaction between daunomycinone of formula (II) and a halide compound of formula (III) may be carried out in the same manner as in the process according to the third aspect of this invention. In this case, the subsequent conversion of the l4-methyl group into a hydroxymethyl group is carried out in the following manner.

Firstly, the l4-methyl group is brominated with bromine. Organic solvent to be used for said bromination reaction may be halogenated hydrocarbons such as dichloromethane; lower alkanols such as methanol and ethanol; and ethers such as dioxane and tetrahydrofuran. The bromination reaction may be conducted at a temperature in the range of 0 ° C-50 ° C. It is desirable for said bromination reaction to adopt Arcamone's method in which the reaction is effected in the presence of an alkyl orthoformate (refer to Japanese Patent Publication No. 369l9/82). In such a case, the l3-carbonyl group appears to be protected by being converted in the form of dimethylketal. The brominated product (of the ketal type at the l3-carbonyl group) thus

obtained (i.e. the 14-bromo-substituted product) is then decomposed with an acid or acetone to restore the original ketone group (i.e. the 13-carbonyl group). When acetone is used for this purpose, the acetone is reacted with the 3- and 4-hydroxyl groups on the talose moiety of the 14-bromo-substituted product to form 3′,4′-O-isopropylidene derivative, which is then treated with an acid to remove the isopropylidene group. Then, the bromo-substituent on the 14-methyl group is converted into a hydroxyl group by the action of sodium formate. If, in such conversion step, the 14-O-formyl group is occasionally formed by side reaction, the O-formyl group is converted into hydroxyl group by treatment with a weak acid or weak alkali (refer to Japanese Patent Publication No. 36919/82).

In cases where the compounds of formula (Ia) contains hydroxyl-protecting groups (A), the said protecting groups (A) may first be removed if desired, and then the 14-methyl group may be oxidized into the hydroxymethyl group. Alternatively, the 14-methyl group of said compounds of formula (Ia) may first be oxidized and then the remaining hydroxyl-protecting group (A) may be removed. The removal of the hydroxyl-protecting groups (A) may be effected by hydrolysis in a usual manner.

2,6-Dideoxy-2-fluoro-α-L-talopyranosyl halides of formula (III) above to be used in the processes according to this invention are novel compounds per se and may be prepared, for example, as follows:

Methyl 2,6-dideoxy-2-fluoro-α-L-talopyranoside of formula (h) above which is also a novel compound and which may, for example, be, and which has actually been, prepared by the process as described in Example 1, (10) hereinafter given is used as starting compound for this purpose. This compound is first reacted with acetic anhydride in the presence of sulfuric acid to form 1,3,4-tri-O-acetyl-2,6-dideoxy-2-fluoro-α-L-talopyranose of formula (ℓ)

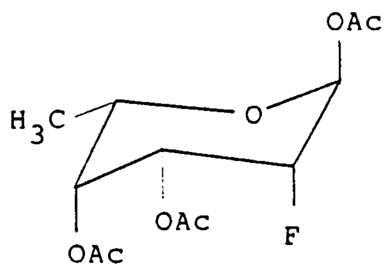

(ℓ)

wherein Ac is acetyl. The compound of formula (ℓ) is then reacted with a titanium tetrahalide such as titanium tetrabromide, titanium tetrachloride or titanium tetraiodide in an inert organic solvent such as dichloromethane, ethyl acetate or preferably a mixture thereof under anhydrous conditions at room temperature or under heating, or alternatively with hydrogen bromide or hydrogen chloride in the form of a solution in acetic acid, whereby to form a 3,4-di-O-acetyl-2,6-dideoxy-2-fluoro-α-L-talopyranosyl halide of formula (m)

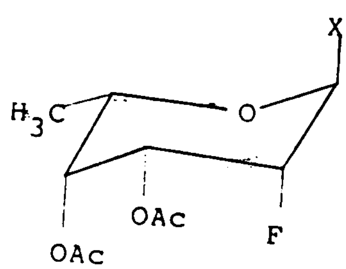

(m)

wherein Ac is acetyl and X is chlorine, bromine or iodine atom. Finally, the acetyl groups of the resulting compound of formula (m) is removed in an inert solvent to give the desired compound of formula (III). The removal of acetyl groups from the compound of formula (m) may, for example, be effected by reacting the said compound with an aqueous hydrobromic acid solution. In cases where the compounds of formula (III) are 3,4-di-O-protected derivatives of 2,6-dideoxy-2-fluoro-α-L-talopyranosyl halides, such derivatives may typically be the compound of formula (m), that is the compound of formula (III) where each A is acetyl as the hydroxyl-protecting group.

Compounds of formula (III) where each A is various, other acyl groups than acetyl as hydroxyl-protecting group can be pepared by the process explained above except that the first step for preparing the

compound of formula (ℓ) from the compound of formula (h) is modified by using other lower alkanoic acid anhydrides or chlorides or aromatic carboxylic acid anhydrides or chlorides such as benzoic acid anhydride or chloride in place of the acetic anhydride to be reacted with the compound of formula (h) so that the hydroxyl groups at the l-, 3- and 4-positions of the compound of formula (h) are protected by the corresponding acyl groups.

The novel compound, methyl 2,6-dideoxy-2-fluoro-α-L-talopyranoside of formula (h) takes a form of crystals with mp. of ll2 - ll4° C and specific rotation $[\alpha]_D^{25}$ of -l24° (c l, methanol).

The following Examples concretely illustrate the production of typical anthracycline derivatives of formula (I) according to this invention and the preparation of a series of intermediates to be used for the preparation of the compounds of formula (I) starting from L-fucose. Thus, Example l illustrates the preparation of methyl 2,6-dideoxy-2-fluoro-α-L-talopyranoside of formula (h) starting from L-fucose, Example 2 is the preparation of 3,4-di-O-acetyl-2,6-dideoxy-2-fluoro-α-L-talopyranosyl bromide of formula (i) and Examples 3 and 4 are the preparation of the compounds of formula (I) according to this invention.

Example l Preparation of methyl 2,6-dideoxy-2-fluoro-α-L-talopyranoside

(l) Methyl 6-deoxy-3,4-O-isopropylidene-α-L-galactopyranoside

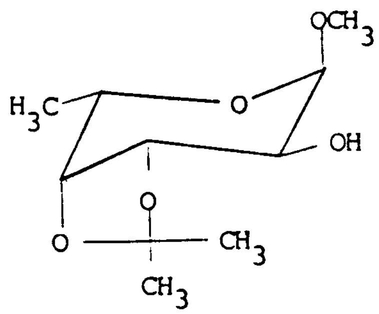

A suspension of L-fucose (2.90 g) in methanol (40 ml) containing l% hydrogen chloride was heated for 8 hours under reflux. The resulting homogeneous solution was cooled to room temperature, neutralized with the addition of basic lead carbonate and then filtered. The filtrate was concentrated to give a colorless solid (3.04 g) comprising mixture of methyl fucosides. The solid was dissolved in dry dimethylformamide (40 ml), to which were added 2,2-dimethoxypropane (7.8l g) and anhydrous p-toluenesulfonic acid (870 mg) and the reaction was conducted at room temperature for 2 hours. The reaction solution was neutralized with sodium hydrogen carbonate and then filtered to remove the insoluble matters. The filtrate was concentrated in vacuo and the residue obtained was dissolved in chloroform (l00 ml). The resulting solution was washed with a saturated aqueous sodium hydrogen carbonate solution and then with an aqueous l0% sodium chloride solution and then concentrated. The residue was treated by silica gel column chromatography on a column of silica gel (400 ml) using a mixture of hexane and acetone (2:l by volume) as eluent (750-l200 ml), to isolate and purify the titled compound as a syrup. Yield: 2.28 g (59%).

$[\alpha]_D^{26}$ - l54° (c l, chloroform)

$^1$H-NMR spectrum (deuterochloroform):

δ 4.72 (lH, d, H-l) $J_{1,2}$ 3.5 Hz

(2) Methyl 2-O-acetyl-6-deoxy-3,4-O isopropylidene-α-L-galactopyranoside

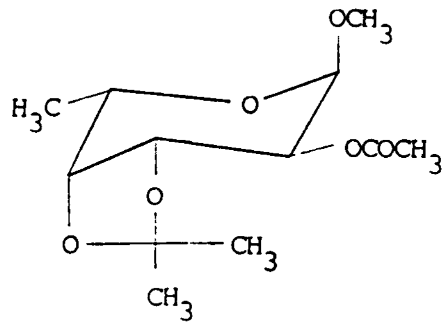

Methyl 6-deoxy-3,4-O-isopropylidene-α-L-galactopyranoside (l2.56 g) was dissolved in dry pyridine (35 ml) and acetic anhydride (l7 ml) was added to the solution. The reaction was conducted at room temperature for 8 hours, after which water (20 ml) was added to the resulting reaction solution, and the mixture obtained was concentrated in vacuo. The residue was dissolved in chloroform (500 ml) and the solution was washed, successively, with an aqueous l0% potasssium hydrogen sulphate solution, a saturated aqueous sodium hydrogen carbonate solution and water and then concentrated to yield the titled compound (l3.8l g; 92%) as colorless crystals. Recrystallization from a mixture of ethyl ether and hexane afforded needle crystals.

m.p. l0l - l02 ° C

$[\alpha]_D^{26}$ - l76 ° (c l, chloroform)

$^1$H-NMR spectrum (deuterochloroform)

δ 4.92 (lH, dd, H-2)

4.79 (lH, d, H-l)

| Elemental analysis ($C_{12}H_{20}O_6$): | | |
|---|---|---|
| Calculated: | C 55.37; | H 7.74% |
| Found: | C 55.27; | H 7.80% |

(3) Methyl 2-O-acetyl-6-deoxy-α-L-galactopyranoside

Methyl 2-O-acetyl-6-deoxy-3,4-O-isopropylidene-α-L-galactopyranoside (l3.8l g) obtained as above was dissolved in an aqueous 80% acetic acid solution (l40 ml) and the reaction was conducted at 80 ° C for l hour. The reaction solution was concentrated in vacuo and the resultant residue was treated by silica gel colum chromatography on a silica gel column (600 ml) using a mixture of hexane and acetone (l:2 by volume) as eluent(ll00-l950 ml), to isolate and purify the titled compound as colorless crystals (ll.l7 g; 96%). Recrystallization was made from an ethyl ether-hexane mixture.

m p. 77 - 78 ° C

$[\alpha]_D^{26}$ -l82 ° (c 2, chloroform)

(4) Methyl 2-O-acetyl-6-deoxy-3-O-tosyl-α-L-galactopyranoside

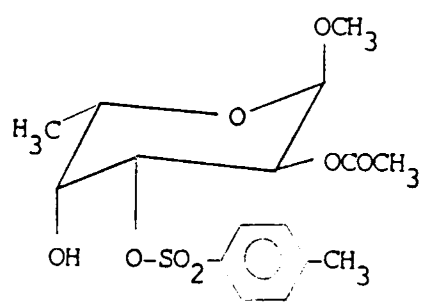

Methyl 2-O-acetyl-6-deoxy-α-L-galactopyranoside (ll g) was dissolved in dry pyridine (200 ml), and to the resulting solution, after cooled to -20 ° C, was added p-toluenesulfonyl chloride (l3.33 g). The reaction was conducted at -20 ° C for 26 hours and then at room temperature for l9 hours. The reaction solution, after water ( 5 ml) was added thereto, was concentrated in vacuo. The residue was treated by silica gel

column chromatography on silica gel column (700 ml) using a mixture of hexane and acetone (l:l by volume as eluent (l550-2600 ml), to isolate and purify the titled compound as colorless crystals (l6.25 g; 87%). Recrystallization was effected from an ethyl ether-hexane mixture.

mp. ll8 - l20 ° C

$[\alpha]_D^{26}$ - l36 ° (c l, chloroform)

$^1$H-NMR spectrum (deuterochloroform):

δ 5.l6 (lH, dd, H-2)

4.94 (lH, dd, H-3)

4.87 (lH, d, H-l)

2.45 (3H, s, $CH_3$ in tosyl)

l.79 (3H, s, Acetyl)

| Elemental analysis ($C_{16}H_{22}O_8S_1$) | | | |
|---|---|---|---|
| Calculated: | C 5l.33; | H 5.92; | S 8.56% |
| Found: | C 5l.4l; | H 6.06; | S 8.65% |

(5) Methyl 2-O-acetyl-4-O-benzyl-6-deoxy-3-O-tosyl-α-L-galactopyranoside

Methyl 2-O-acetyl-6-deoxy-3-O-tosyl-α-L-galactopyranoside (l60 mg) was dissolved in a mixture (3.2 ml) of cyclohexane and dichloromethane (2:l by volume), to which were then added benzyl 2,2,2-trichloroacetimidate

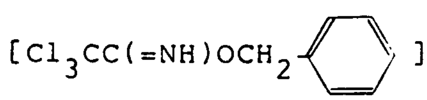

(2l4 mg) and trifluromethanesulfonic acid (0.0l5 ml), and the whole mixture was kept at room temperature for 2 hours to effect the reaction. The reaction solution was diluted with chloroform, then washed with a saturated aqueous sodium hydrogen carbonate solution and then with water and finally concentrated. The residue was treated by silica gel column (30 ml) chromatography using a mixture of toluene and ethyl acetate (6:l by volume, 55-80 ml), to isolate and purify the titled compound as a syrup (l64 mg; 83%).

$[\alpha]_D^{26}$ - l0l ° (c l.5, chloroform)

(6) Methyl 2,3-anhydro-4-O-benzyl-6-deoxy-α-L-gulopyranoside

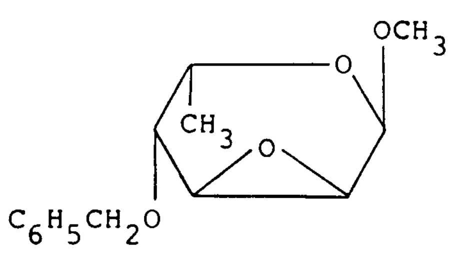

Methyl 2-O-acetyl-4-O-benzyl-6-deoxy-3-O-tosyl-α-L-galactopyranoside (l9.72 g) obtained as above was dissolved in dry methanol (400 ml), to which a 28% methanolic solution of sodium methoxide (l23 ml) was added, and the resulting mixture was kept at room temperature for 4.5 hours to effect the reaction. The reaction solution, after carbon dioxide was introduced thereinto, was concentrated, and the residue was dissolved in chloroform (300 ml). The solution in chloroform was washed with water and then concentrated. The residue was treated by silica gel column (800 ml) chromatography using a mixture of hexane and acetone (3:l by volume) as eluent (l350-2550 ml), to isolate and purify the titled compound as a colorless syrup (6.62 g; 62%).

$[\alpha]_D^{26}$ - 25 ° (c 3, chloroform)

(7) Methyl 4-O-benzyl-2,6-dideoxy-2-fluoro-α-L-idopyranoside

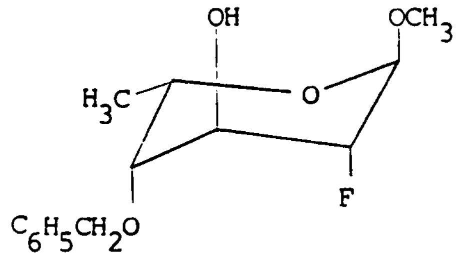

Methyl 2,3-anhydro-4-O-benzyl-6-deoxy-α-L-gulopyranoside (l40 mg) was dissolved in dry ethylene glycol (2.8 ml), to which potassium hydrogen fluoride ($KHF_2$) (880 mg) was then added, and the resultant mixture was stirred at l80 °C for 3 hours. The reaction solution obtained was diluted with chloroform, washed with a saturated aqueous sodium hydrogen carbonate and then with water and concentrated. The residue was treated by silica gel column (30 ml) chromatography using a mixture of hexane and acetone (3:l by volume) as eluent 75-l05 ml), to isolate and purify the titled compound as a syrup (67 mg; 44%).
$[\alpha]_D^{26}$ - 62 ° (c 2, chloroform)
$^1$H-NMR spectrum (deuterochloroform):
δ 4.79 (lH, dd, H-l)
4.32 (lH, dddd, H-2)
$^{19}$F-NMR spectrum (deuterochloroform; $CFCl_3$ as internal standard):
Φ - l96.0 (ddd) $J_{F,H-2}$ 48, $J_{F,H-3}$ ll, $J_{F,H-1}$ 9 Hz

| Elemental analysis ($C_{14}H_{19}O_4F_1$) | | | |
|---|---|---|---|
| Calculated: | C 62.2l; | H 7.08; | F 7.03% |
| Found: | C 6l.98; | H 7.l7; | F 7.0l% |

(8) Methyl 4-O-benzyl-2,6-dideoxy-2-fluoro-α-L-lyxohexopyranosid-3-ulose

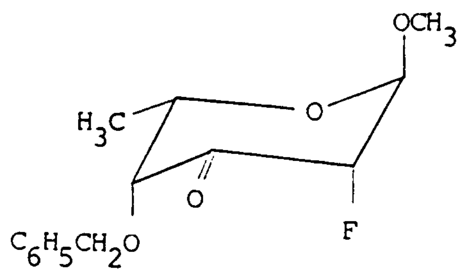

Methyl 4-O-benzyl-2,6-dideoxy-2-fluoro-α-L-idopyranoside (l39 mg) obtained as above was dissolved in a mixture of dry benzene (l ml) and dry dimethylsulfoxide (0.l4 ml) which acts as both a solvent and an oxidizing agent. To the resulting solution were added dicyclohexylcarbodiimide (l55 mg), pyridine (0.0l ml) and pyridinium trifluoroacetate (23 mg), and the resulting mixture was stirred at room temperature for 3 hours to effect the reaction. The reaction solution was admixed with a methanolic solution of oxalic acid (l42 mg) to decompose the excess of the dicyclohexylcarbodiimide. Then, the reaction solution so treated was diluted with benzene (30 ml) and filtered to remove the insolubles. The filtrate was washed with a saturated aqueous sodium hydrogen carbonate solution and then with water, and concentrated. The residue was treated by silica gel column (25 ml) chromatography using a mixture of hexane and acetone (3:l by volume) as eluent (40-70 ml), to isolate and purify the titled compound as needle crystals (ll0 mg; 79%).
mp. 63 - 64 °C
$[\alpha]_D^{26}$ - 7 ° (c l, chloroform)
$^1$H-NMR spectrum (deuterochloroform)
δ 4.80 (lH, dd, H-l)
δ 4.66 (lH, ddd, H-2)

(9) Methyl 4-O-benzyl-2,6-dideoxy-2-fluoro-α-L-talopyranoside

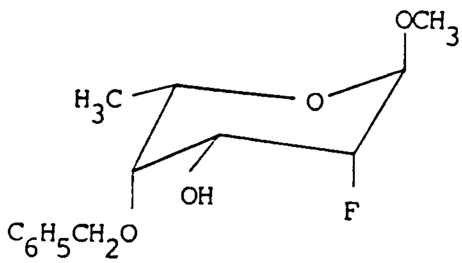

Methyl 4-O-benzyl-2,6-dideoxy-2-fluoro-α-L-lyxohexopyranosid-3-ulose (698 mg) was dissolved in dry tetrahydrofuran (l4 ml), and to the resulting solution, after cooled to -30°C, was added a suspension of lithium aluminium hydride (l98 mg) in dry tetrahydrofuran (2 ml). The mixture was stirred at -30°C for 45 minutes, then at -l0°C for 2 hours and finally at 0°C for 30 minutes. A saturated aqueous ammonium chloride solution was then added to the resultant reaction solution as cooled at 0°C, to which chloroform (50 ml) was further added. The resulting mixture was filtered. The chloroform solution thus separated was washed with water and concentrated. The residue was treated by silica gel column (l00 ml) chromatography using a mixture of hexane and acetone (3:l by volume) as eluent, to isolate and purify the titled compound as a thick syrup (576 mg; 82%).

$[\alpha]_D^{26}$ - 98° (c 3.5, chloroform)

$^{19}$F-NMR spectrum (deuterochloroform; $CFCl_3$ internal standard):

Φ - 206.0 (ddd) $J_{F,H-2}$ 49.5, $J_{F,H-3}$ 3l.5, $J_{F,H-1}$ 9 Hz

(l0) Methyl 2,6-dideoxy-2-fluoro-α-L-talopyranoside

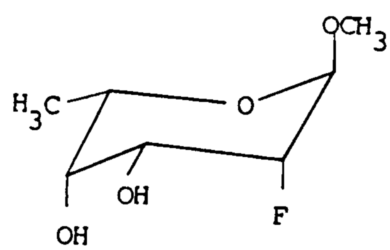

Methyl 4-O-benzyl-2,6-dideoxy-2-fluoro-α-L-talopyranoside (345 mg) obtained as above was dissolved in a mixture (8 ml) of dioxane, acetic acid and water (l0:l:l by volume), and the resulting solution was subjected to catalytic reduction with hydrogen at atmospheric pressure in the presence of palladium black catalyst to remove the benzyl group for the deprotection. The reaction mixture obtained was filtered and the filtrate was concentrated in vacuo to yield a colorless solid (230 mg). Recrystallization of this solid from a mixture of chloroform and hexane gave the titled compound as colorless crystals (l86 mg; 8l%).

mp. ll2-ll4°C

$[\alpha]_D^{23}$ - l24° (c l, methanol)

$^{1}$H-NMR spectrum (deuterochloroform):

δ 4.87 (lH, dd, H-l)

δ 4.58 (lH, ddt, H-2)

$^{19}$F-NMR spectrum (deuterochloroform; internal standard: $CFCl_3$)

Φ - 203.l (dddd) $J_{F,H-2}$ 49, $J_{F,H-3}$ 32, $J_{F,H-l}$ 9. $J_{F,OH-4}$ 7.5 Hz.

Example 2

Preparation of 3,4-di-O-acetyl-2,6-dideoxy-2-fluoro-α-L-talopyranosyl bromide

(l) l,3,4-Tri-O-acetyl-2,6-dideoxy-2-fluoro-α-L-talopyranose

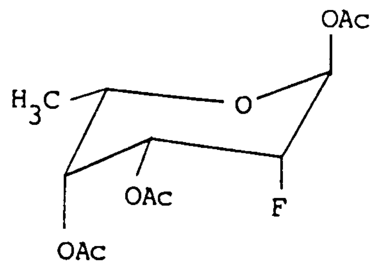

Ac = acetyl

Methyl 2,6-dideoxy-2-fluoro-α-L-talopyranoside (230 mg) which was prepared as in Example I (I0) was dissolved in dry nitromethane (7.6 ml), to which were added acetic anhydride (I.3 ml) and sulfuric acid (0.036 ml). The resulting mixture was kept at room temperature for 4 hours to effect the acetylation. The reaction solution was neutralized with a saturated aqueous sodium hydrogen carbonate solution and then diluted with chloroform (50 ml). The diluted solution was washed with water and concentrated. The residue was treated by silica gel column (60 ml) chromatography using a mixture of hexane and acetone (3:I by volume) as eluent(II0-I75ml), to isolated and purify the titled compound as colorless crystals (3I3 mg; 84%). The product was recrystallized from a mixture of ether and hexane.
mp. I02 - I03 ° C
$[\alpha]_D^{26}$ - III ° (c I, chloroform)
$^1$H-NMR spectrum (deuterochloroform):
δ 6.33 (IH, dd, H-I)
4.55 (IH, dddd, H-2)

| Elemental analysis ($C_{12}H_{17}O_7F$) | | | |
|---|---|---|---|
| Calculated: | C 49.32; | H 5.86; | F 6.50% |
| Found: | C 49.I9; | H 6.00; | F 6.39% |

(2) 3,4-di-O-acetyl-2,6-dideoxy-2-fluoro-α-L-talopyranosyl bromide

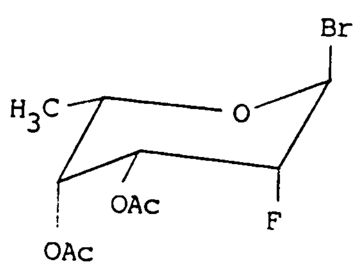

Ac = acetyl

I,3,4-Tri-O-acetyl-2,6-dideoxy-2-fluoro-α-L-talopyranose (327 mg) obtained as above was dissolved in a mixture (7 ml) of dry dichloromethane and dry ethyl acetate (I0:I by volume), to which titanium tetrabromide (534 mg) was added, and the mixture was allowed to stand at room temperature for 22 hours for the reaction. To the reaction solution, there were added, succesively, dry acetonitrile (I0 ml), dry sodium acetate (I.67 g) and dry toluene (20 ml). The precipitate thus deposited was removed by filtration and the filtrate was concentrated in vacuo. Dry toluene (20 ml) was added to the residue, and the mixture was filtered to remove the insolubles. The filtrate was concentrated in vacuo to afford the titled compound as a syrup (330 mg; 94%).
$[\alpha]_D^{25}$ - I54 ° (c I, chloroform)
$^1$H-NMR spectrum (deuterochloroform):
δ 6.55 (IH, broad d, H-I)
4.8I (IH, ddt, H-2)

Example 3

Production of 7-O-(2,6-dideoxy-2-fluoro-α-L-talopyranosyl)daunomycinone

(l) 7-O-(3,4-di-O-acetyl-2,6-dideoxy-2-fluoro-α-L-talopyranosyl) daunomycinone

To a suspension of daunomycinone (290 mg), mercuric oxide (yellow color) (943 mg), mercuric bromide (273 mg) and "Molecular Sieves" 3A in powder form (4.5 g) in dry dichloromethane (36 ml), there was added a solution of 3,4-di-O-acetyl-2,6-dideoxy-2-fluoro-α-L-talopyranosyl bromide (330 mg) as prepared in Example 2(2), in dry dichloromethane (9 ml). The resulting mixture was stirred in a dark place at room temperature for 20 hours and filtered. The filtrate was diluted with chloroform and the diluted solution was washed with an aqueous 30% potassium iodide solution, a saturated aqueous sodium hydrogen carbonate solution and water, successively, and concentrated. The residue was treated by silica gel column (60 ml) chromatography using a mixture of benzene and acetone (4:l by volume) as developing solvent(l25-2l0 ml), to isolate and purify the titled compound as a red solid (378 mg; 82%). This product was reprecipitated from a mixture of chloroform and hexane.

mp. l44 - l46 °C

$[\alpha]_D^{26}$ + 2ll ° (c 0.036, chloroform)

$^1$H-NMR spectrum (deuterochloroform):

δ 5.64 (lH, dd, H-l′)

4.08 (3H, s, $OCH_3$)

2.4l (3H, s, Acetyl)

2.l8, 2.03 (each 3H, s, OAc)

$^{19}$F-NMR spectrum (deuterochloroform internal standard: $CFCl_3$):

Φ -20l.0 (ddd) $J_{F,H\text{-}2'}$ 49.5, $J_{F,H\text{-}3'}$ 32.5, $J_{F,H\text{-}l'}$ 9.5 Hz

| Elemental analysis ($C_{31}H_{31}O_{13}F \bullet H_2O$) | | | |
|---|---|---|---|
| Calculated: | C 57.4l; | H 5.l3; | F 2.93% |
| Found: | C 57.77, | H 5.28; | F 3.2l% |

(2) 7-O-(2,6-dideoxy-2-fluoro-α-L-talopyranosyl) daunomycinone

7-O-(3,4-Di-O-acetyl-2,6-dideoxy-2-fluoro-α-L-talopyranosyl) daunomycinone (l00 mg) obtained as above was dissolved in 0.2 N aqueous sodium hydroxide solution (8 ml), and the solution was allowed to cause hydrolysis at 0 °C for 5 hours, whereby to remove the acetyl groups. The reaction solution obtained was neutralized at that temperature with the addition of l N hydrochloric acid (l.6 ml), after which sodium chloride (l.5 g) was added thereto, and the mixture was extracted with chloroform. The extract in chloroform was washed with a saturated aqueous sodium chloride solution and then concentrated. The red solid residue obtained was reprecipitated from a mixture of chloroform and hexane to yield the titled compound as a red solid (62 mg; 72%).

$[\alpha]_D^{25}$ + l97 ° (c 0.02, chloroform-methanol = l:l)

$^1$H-NMR spectrum (deuteropyrdine):

δ 6.02 (lH, broad d, H-l′)

3.98 (3H, s, $OCH_3$)

δ 2.57 (3H, s, Acetyl)

Example 4

Production of 7-O-(2,6-dideoxy-2-fluoro-α-L-talopyranosyl)adriamycinone

7-O-(2,6-dideoxy-2-fluoro-α-L-talopyranosyl) daunomycinone (37.8 mg) prepared as in Example 3 was suspended in a mixture of dry methanol (0.9 ml) and dry dioxane (l.4 ml). Then, methyl orthoformate (0.052 ml) was added to the resultant suspension for the reaction to protect the l3-carbonyl group of the daunomycinone derivative by ketalization into the form of the dimethylketal. Thereafter, the resulting reaction mixture was cooled to 0 °C, followed by adding to the suspension a solution of bromine (l5 mg) in dry dichloromethane (0.l5 ml). The resulting mixture was stirred at 0 °C for l hour and then at room temperature for l.5 hours, whereby to brominate the methyl group at the l4-position of the daunomycinone derivative.

The resulting homogeneous solution was added dropwise to isopropylether (l2 ml), and red precipitate

thus formed was recovered by centrifuging and washed twice with isopropylether. The precipitate was then suspended in acetone (3 ml) and the suspension was stirred at room temperature for 40 minutes to conduct the deketalization reaction. Concurrently, the acetone reacted with the daunomycinone derivative to give the 3',4'-O-isopropylidene derivative therefrom. To the homogenous solution thus formed were added isopropylether (5 ml) and hexane (20 ml), and the precipitate deposited was recovered by centrifuging to afford a red solid (35 mg) mainly comprising 7-O-(2,6-dideoxy-2-fluoro-3,4-O-isopropylidene-α-L-talopyranosyl)-14-bromodaunomycinone. The red solid obtained was dissolved in a mixture of acetone (3.2 ml) and water (0.8 ml), to which sodium formate (65 mg) was added. The mixture obtained was vigorously stirred at room temperature for 17 hours, whereby to convert the bromo group at the 14-position of the danomycinone derivative into the hydroxyl group including the formyloxy group. The reaction solution obtained was concentrated to a small volume and the solid thus deposited was washed with water and dried to afford a red solid (29 mg). This solid dissolved in chloroformmethanol (1:1, 3 ml) containing aqueous 1 M ammonia (0.37 ml) was kept at 0°C for 40 minutes (the formyl group as by-formed was removed by this procedure). After concentration, the residue was dissolved in a 80% aqueous acetic acid (1.4 ml), and the solution thus formed was heated at 80°C for 1.5 hours. This heating served to remove the 3',4'-O-isopropylidene group which had been introduced by the acetone treatment as above. The reaction solution so obtained was concentrated in vacuo, and water was added to the residue. The resulting mixture was centrifuged to collect the solid material which was then washed with water. The solid material so obtained was reprecipitated from a mixture of chloroform, methanol and isopropylether (5:l:40 by volume) to afford the titled compound as a red solid (l6.7 mg; Yield 43%). The water washings above were passed through a column of 3 ml of Diaion HP-50 resin(Diaion is a registered trade mark of a microporous, adsorbent resin as produced by Mitsubishi Chemical Industries, Ltd.). The resin column was washed with water and then eluted with aqueous 80% methanol, namely a mixture of methanol and water (4:l by volume). Fractions containing the titled compound (fraction Nos. 3-2l fraction, l5-l05 ml) were collected, combined together and concentrated to afford a second crop (5 mg) of the titled compound as a red solid. The total yield of the desired compound was 2l.7 mg (56%).

$[\alpha]_D^{25}$ + l94° (c 0.0l, chloroform-methanol (l:l by volume))

[1]N-NMR spectrum (pyridine-$d_5$):

δ 5.95 (lH, broad d, H-l')

5.33 (2H, s, $CH_2OH$)

3.96 (3H, s, $O\overline{C}H_3$)

**Claims**

**Claims for the following Contracting States : CH, DE, FR, GB, IT, LI, NL,**

1. An anthracycline derivative represented by the general formula

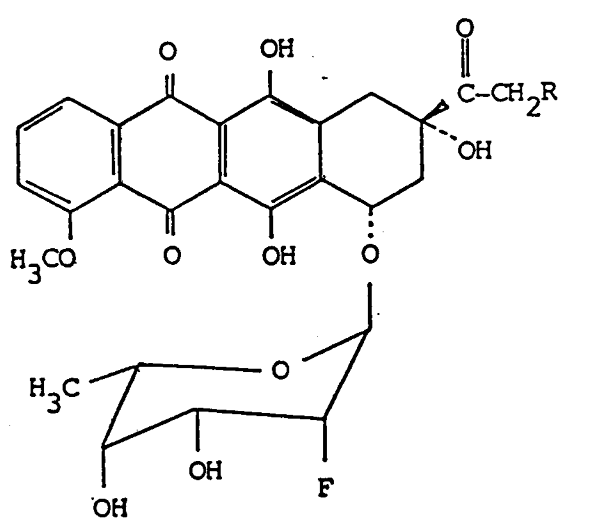

(I)

wherein R is a hydrogen atom or a hydroxyl group.

2. A compound as claimed in Claim 1, which is 7-O-(2,6-dideoxy-2-fluoro-α-L-talopyranosyl)-

daunomycinone.

**3.** A compound as claimed in Claim 1, which is 7-O-(2,6-dideoxy-2-fluoro-α-L-talopyranosyl)-adriamycinone.

**4.** A pharmaceutical antitumor composition comprising an anthracycline derivative represented by the general formula

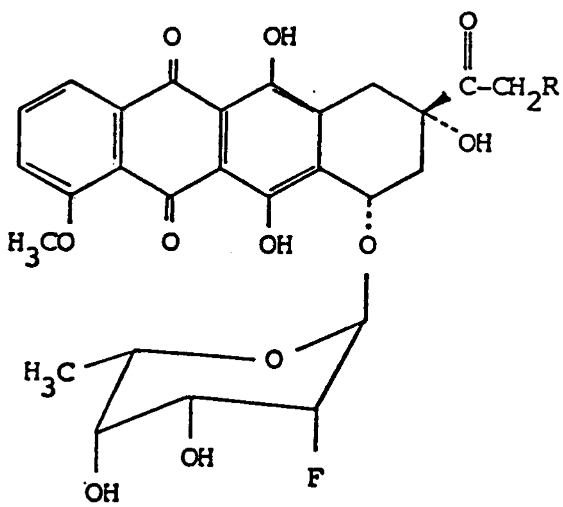

(I)

wherein R is a hydrogen atom or a hydroxyl group, as the active ingredient, in association with a pharmaceutically acceptable carrier for the active ingredient.

**5.** A process for the production of an anthracycline derivative represented by the formula

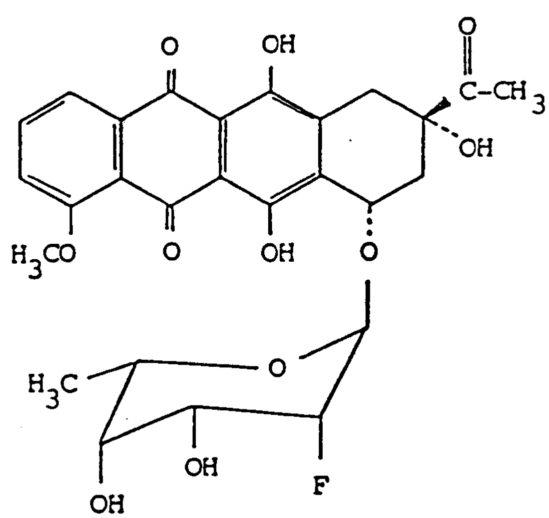

(Ib )

which comprises reacting daunomycinone of the formula

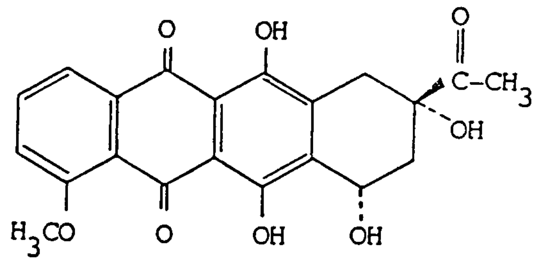

(II)

with a 2,6-dideoxy-2-fluoro-α-L-talopyranosyl halide of the formula

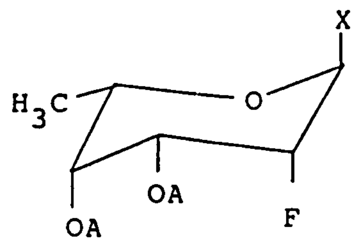

(III)

wherein X is a bromine, iodine or chlorine atom and A is a hydroxyl-protecting group or a hydrogen atom, to produce an anthracycline derivative of the formula

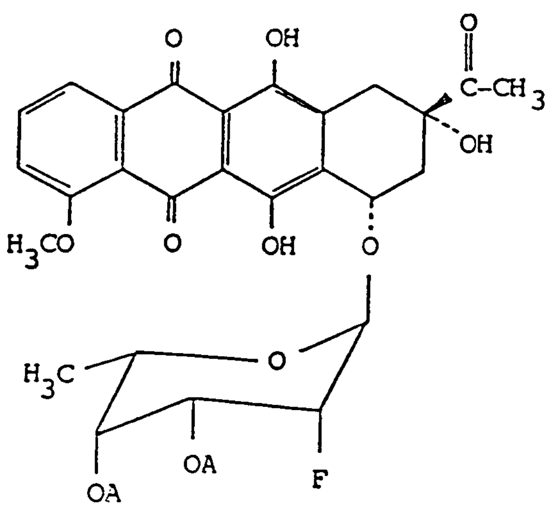

(Ia)

wherein A is as defined above, and then removing the hydroxyl-protecting groups (A) from the compound of the formula (Ia) in a known manner, when the compound of the formula (Ia) is containing the hydroxyl-protecting groups (A) remaining therein.

6. A process as claimed in Claim 5, in which daunomycinone of the formula (II) is reacted with the compound of the formula (III) in an aprotic organic solvent at a temperature of -20 ° C to 80 ° C in the presence of one or more of dehydrohalogenating agents under anhydrous conditions.

7. A process for the production of an anthracycline derivative of the formula

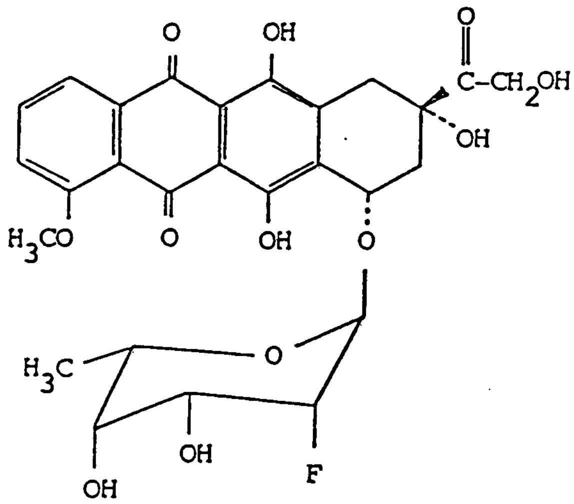

(Id)

which comprises reacting daunomycinone of the formula

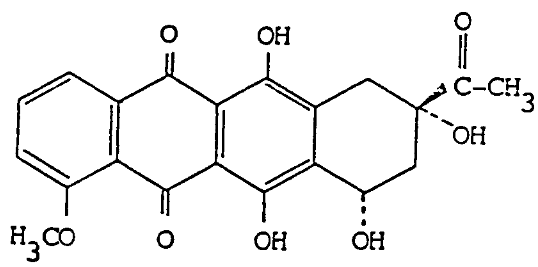

(II)

with a 2,6-dideoxy-2-fluoro-α-L-talopyranosyl halide of the formula

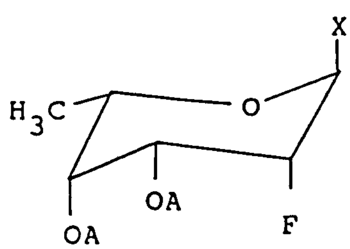

(III)

wherein X is a bromine, iodine or chlorine atom and A is a hydroxyl-protecting group or a hydrogen atom, to produce an anthracycline derivative of the formula

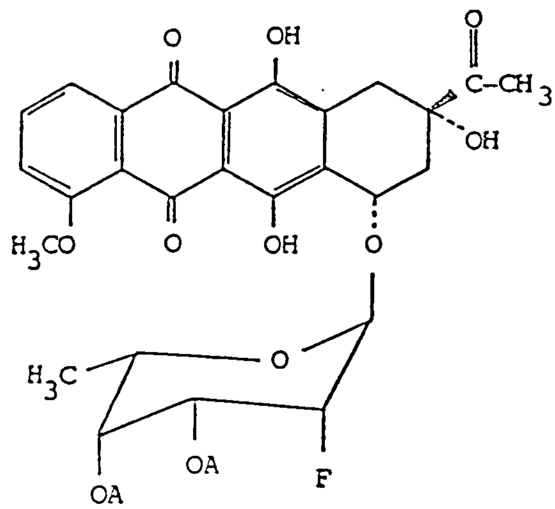

(Ia)

wherein A is as defined above, and then, either, removing the hydroxyl-protecting groups (A) from the compound of the formula (Ia) when the compound of the formula (Ia) is containing the hydroxylprotecting groups (A) remaining therein, to produce the deprotected product having deprived of the hydroxyl-protecting groups (A) and subsequently converting the methyl group at the 14-position of said deprotected product into a hydroxymethyl group to produce the compound of the formula (Id) above, or converting the methyl group at the 14-position of said compound of the formula (Ia) into the hydroxymethyl group to give an anthracycline derivative of the formula

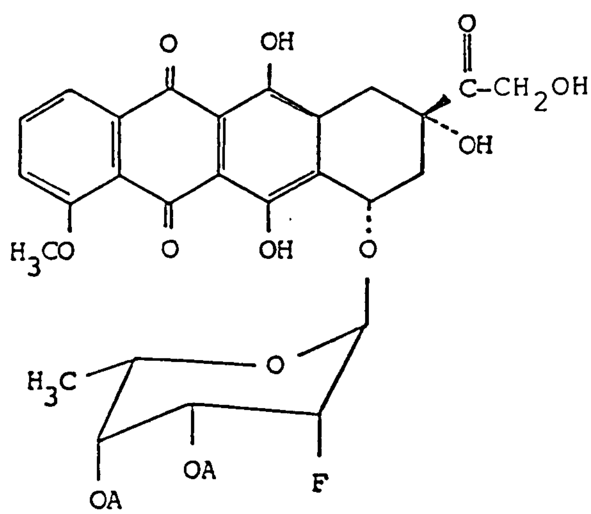

(Ic)

wherein A is as defined above and subsequently removing the hydroxyl-protecting groups (A) from the compound of the formula (Ic) when this compound of the formula (Ic) is containing the hydroxyl-protecting groups (A) remaining therein, to produce the compound of the formula (Id) above.

**8.** A process as claimed in Claim 7, in which daunomycinone of the formula (II) is reacted with the compound of the formula (III) in an aprotic organic solvent at a temperature of -20 °C to 80 °C in the presence of one or more of dehydrohalogenating agents under anhydrous conditions.

**9.** A process as claimed in Claim 7, in which the step of converting the methyl group into the hydroxymethyl group at the 14-position of the deprotected product having deprived of the hydroxyl-protecting groups (A) from the compound of the formula (Ia) or the methyl group at the 14-position of the compound of the formula (Ia) comprises brominating the 14-methyl group into a 14-bromomethyl group by reacting said 14-methyl group with bromine at a temperature of 0 °C to 50 °C in the presence of an alkyl orthoformate which protects the 13-carbonyl group of the anthracycline compound, namely

said deprotected product or the compound of the formula (Ia), and then reacting the 14-(bromomethyl) group so formed with sodium formate to produce the 14-(hydroxy-methyl) group.

**10.** 2,6-Dideoxy-2-fluoro-α-L-talopyranosyl halide of the formula

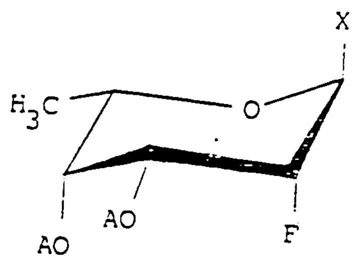

wherein X is a bromine, iodine or chlorine atom and A is a hydroxyl-protecting group or a hydrogen atom.

**11.** A compound as claimed in claim 10 which is 3,4-di-O-acetyl-2,6-dideoxy-2-fluoro-α-L-talopyranosyl bromide.

**12.** Methyl-2,6-dideoxy-2-fluoro-α-L-talopyranoside.

**13.** 1,3,4-tri-O-acetyl-2,6-dideoxy-2-fluoro-α-L-talopyranose.

**Claims for the following Contracting State : ES**

**1.** A process for the production of an anthracycline derivative represented by the formula

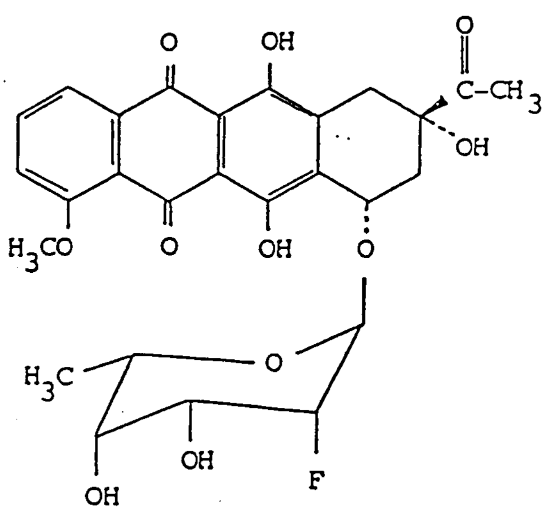

(Ib )

which comprises reacting daunomycinone of the formula

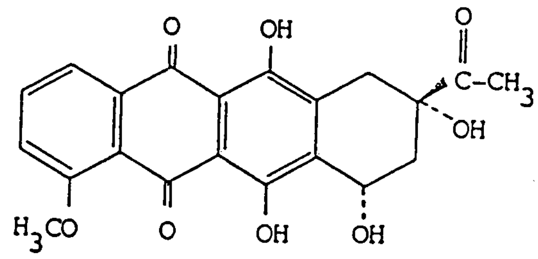

(II)

with a 2,6-dideoxy-2-fluoro-α-L-talopyranosyl halide of the formula

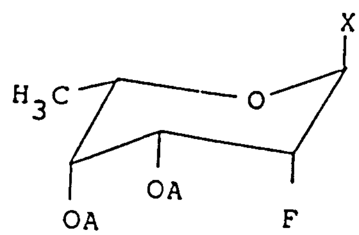

(III)

wherein X is a bromine, iodine or chlorine atom and A is a hydroxyl-protecting group or a hydrogen atom, to produce an anthracycline derivative of the formula

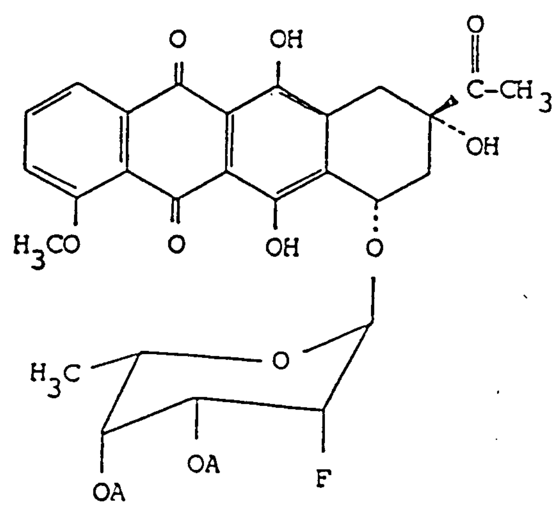

(Ia)

wherein A is as defined above, and then removing the hydroxyl-protecting groups (A) from the compound of the formula (Ia) in a known manner, when the compound of the formula (Ia) is containing the hydroxyl-protecting groups (A) remaining therein.

**2.** A process as claimed in Claim 1 , in which daunomycinone of the formula (II) is reacted with the compound of the formula (III) in an aprotic organic solvent at a temperature of -20 °C to 80 °C in the presence of one or more of dehydrohalogenating agents under anhydrous conditions.

**3.** A process for the production of an anthracycline derivative of the formula

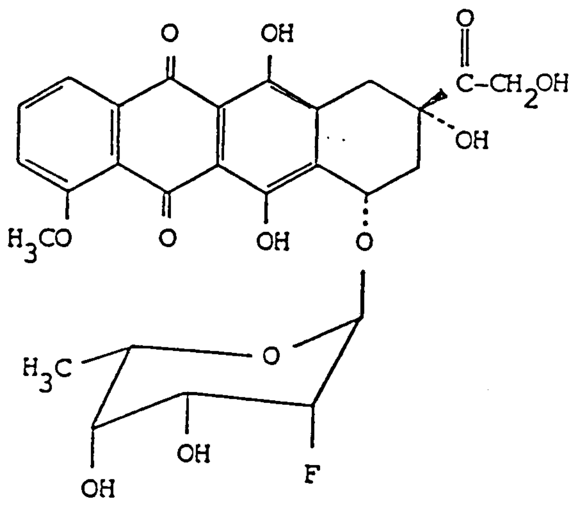

(Id)

which comprises reacting daunomycinone of the formula

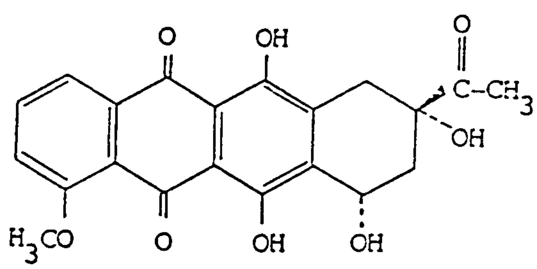

(II)

with a 2,6-dideoxy-2-fluoro-α-L-talopyranosyl halide of the formula

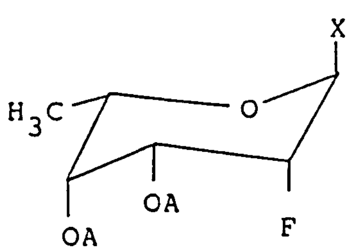

(III)

wherein X is a bromine, iodine or chlorine atom and A is a hydroxyl-protecting group or a hydrogen atom, to produce an anthracycline derivative of the formula

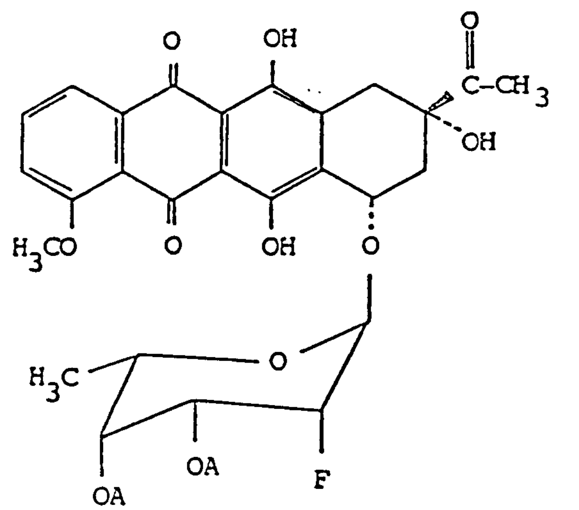

(Ia)

wherein A is as defined above, and then, either, removing the hydroxyl-protecting groups (A) from the compound of the formula (Ia) when the compound of the formula (Ia) is containing the hydroxyl-protecting groups (A) remaining therein, to produce the deprotected product having deprived of the hydroxyl-protecting groups (A) and subsequently converting the methyl group at the 14-position of said deprotected product into a hydroxymethyl group to produce the compound of the formula (Id) above, or converting the methyl group at the 14-position of said compound of the formula (Ia) into the hydroxymethyl group to give an anthracycline derivative of the formula

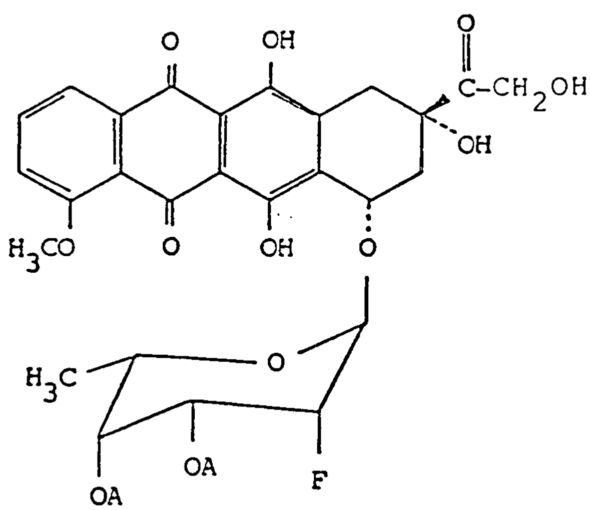

(Ic)

wherein A is as defined above and subsequently removing the hydroxyl-protecting groups (A) from the compound of the formula (Ic) when this compound of the formula (Ic) is containing the hydroxyl-protecting groups (A) remaining therein, to produce the compound of the formula (Id) above.

**4.** A process as claimed in Claim 3, in which daunomycinone of the formula (II) is reacted with the compound of the formula (III) in an aprotic organic solvent at a temperature of -20 °C to 80 °C in the presence of one or more of dehydrohalogenating agents under anhydrous conditions.

**5.** A process as claimed in Claim 3, in which the step of converting the methyl group into the hydroxymethyl group at the 14-position of the deprotected product having deprived of the hydroxyl-protecting groups (A) from the compound of the formula (Ia) or the methyl group at the 14-position of the compound of the formula (Ia) comprises brominating the 14-methyl group into a 14-bromomethyl group by reacting said 14-methyl group with bromine at a temperature of 0 °C to 50 °C in the presence of an alkyl orthoformate which protects the 13-carbonyl group of the anthracycline compound, namely

said deprotected product or the compound of the formula (Ia), and then reacting the 14-(bromomethyl) group so formed with sodium formate to produce the 14-(hydroxy-methyl) group.

6. A process for the production of a pharmaceutical antitumor composition comprising an anthracycline derivative represented by the general formula

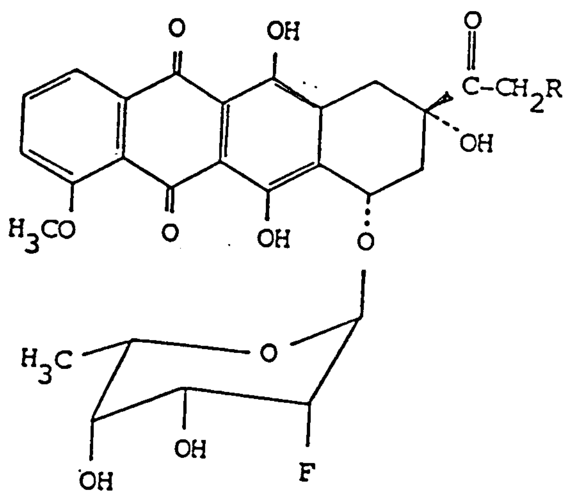

(I)

wherein R is a hydrogen atom or a hydroxyl group, as active ingredient by blending said anthracycline derivative with a pharmaceutically acceptable carrier.

7. A process as claimed in claim 6 wherein the active ingredient is 7-O-(2,6-dideoxy-2-fluoro-α-L-talopyranosyl)-daunomycinone.

8. A process as claimed in claim 6 wherein the active ingredient is 7-O-(2,6-dideoxy-2-fluoro-α-L-talopyranosyl)-adriamycinone.

9. A process for the preparation of 3, 4-di-O-protected-2,6-dideoxy-2-fluoro-α-L-talopyranosyl halide (bromide, iodide or chloride), which comprises reacting 1, 3, 4-tri-O-protected 2,6-dideoxy-2-fluoro-α-L-talopyranose with a titanium tetrahalide.

**Revendications**

**Revendications pour les Etats contractants suivants : CH, DE, FR, GB, IT, LI, NL**

1. Dérivé d'anthracycline représenté par la formule générale :

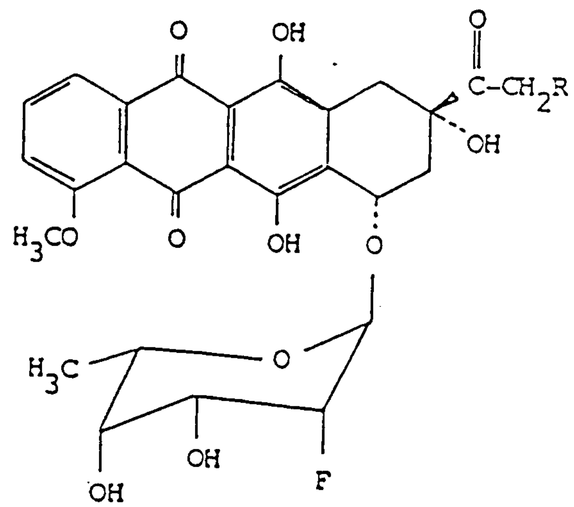

(I)

dans laquelle R est un atome d'hydrogène ou un groupe hydroxyle.

**2.** Composé selon la revendication 1, qui est 7-O-(2,6-didéoxy-2-fluoro-α-L-talopyranosyl)daunomycinone.

**3.** Composé selon la revendication 1, qui est 7-O-(2,6-didéoxy-2-fluoro-α-L-talopyranosyl)adriamycinone.

**4.** Composition pharmaceutique anti-tumorale, contenant un dérivé d'anthracycline représenté par la formule générale :

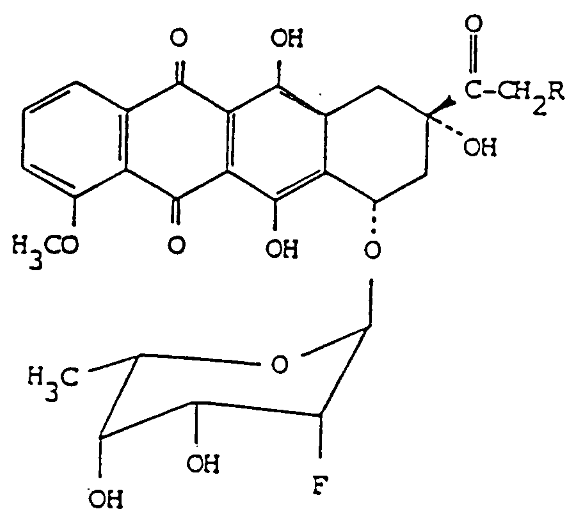

(I)

dans laquelle R est un atome d'hydrogène ou un groupe hydroxyle, en tant qu'ingrédient actif, en association avec un véhicule pharmaceutiquement acceptable pour l'ingrédient actif.

**5.** Procédé pour la préparation d'un dérivé d'anthracycline représenté par la formule :

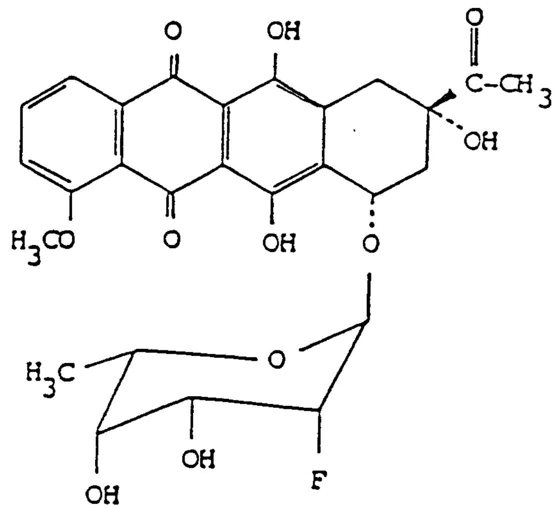

(Ib )

qui consiste à faire réagir une daunomycinone de la formule :

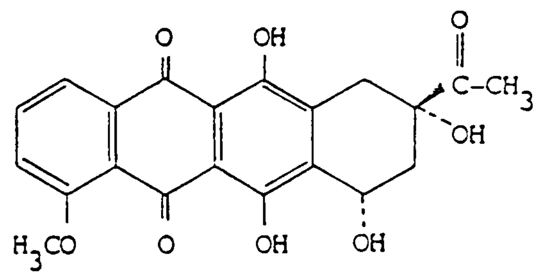

(II)

avec un halogénure de 2,6-didéoxy-2-fluoro-α-L-talopyranosyle de la formule :

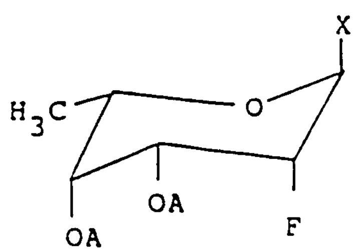

(III)

dans laquelle X est un atome de brome, d'iode ou de chlore, et A est un groupe protecteur d'hydroxyle ou un atome d'hydrogène, pour obtenir un dérivé d'anthracycline de la formule :

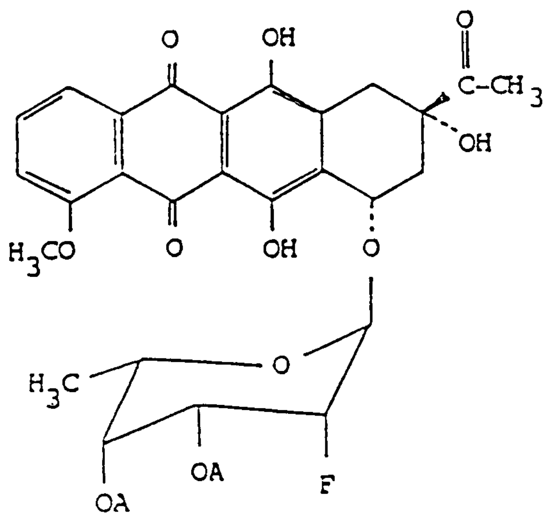

(Ia)

dans laquelle A est comme défini ci-dessus, et à enlever ensuite les groupes protecteurs d'hydroxyle (A) du composé de la formule (Ia) d'une manière connue, lorsque le composé de la formule (Ia) contient des groupes protecteurs d'hydroxyle résiduels (A).

**6.** Procédé selon la revendication 5, dans lequel on fait réagir la daunomycinone de la formule (II) avec le composé de la formule (III) dans un solvant organique aprotique à une température comprise entre -20 ° C et 80 ° C en la présence d'un ou de plusieurs agents de déshydrohalogénation dans des conditions anhydres.

**7.** Procédé pour la préparation d'un dérivé d'anthracycline de la formule :

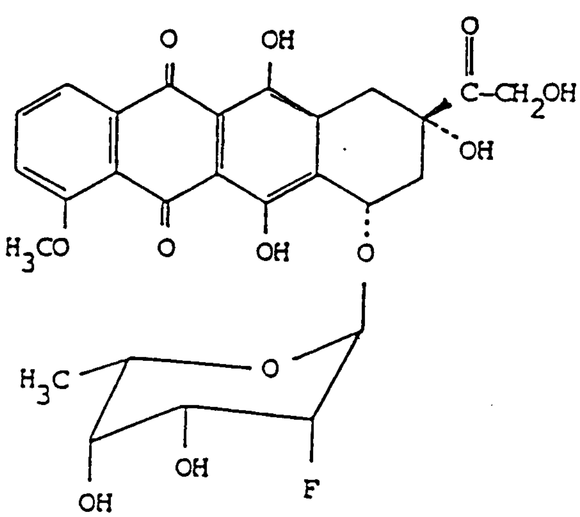

(Id)

qui consiste à faire réagir une daunomycinone de la formule :

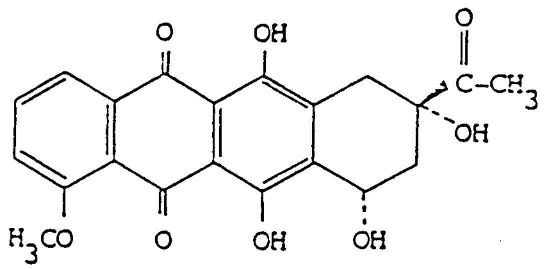

(II)

avec un halogénure de 2,6-didéoxy-2-fluoro-α-L-talopyranosyle de la formule :

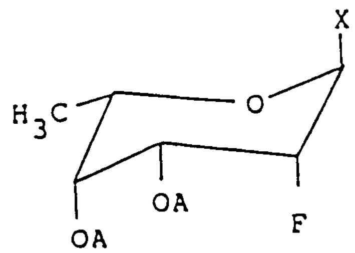

(III)

dans laquelle X est un atome de brome, d'iode ou de chlore, et A est un groupe protecteur d'hydroxyle ou un atome d'hydrogène, pour obtenir un dérivé d'anthracycline de la formule :

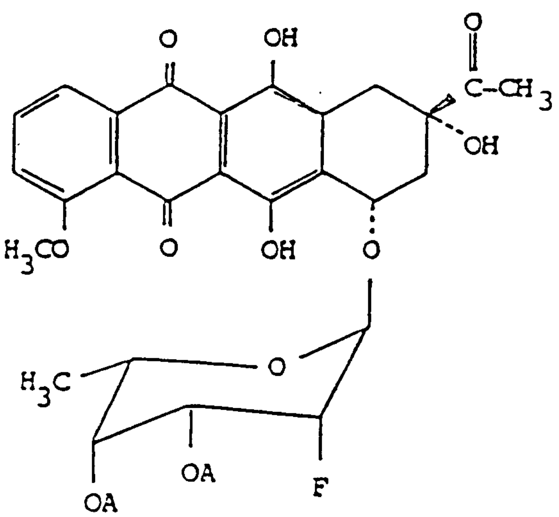

(Ia)

dans laquelle A est comme défini ci-dessus, et ensuite, soit à enlever les groupes protecteurs d'hydroxyle (A) du composé de la formule (Ia) lorsque le composé de la formule (Ia) contient des groupes protecteurs d'hydroxyle (A) résiduels pour produire le produit déprotégé privé des groupes protecteurs d'hydroxyle (A) et à transformer ensuite le groupe méthyle dans la position 14 de ce produit déprotégé en un groupe hydroxyméthyle pour produire le composé de la formule (Id) ci-dessus, soit à transformer le groupe méthyle en position 14 de ce composé de la formule (Ia) en le groupe hydroxyméthyle pour obtenir un dérivé d'anthracycline de la formule :

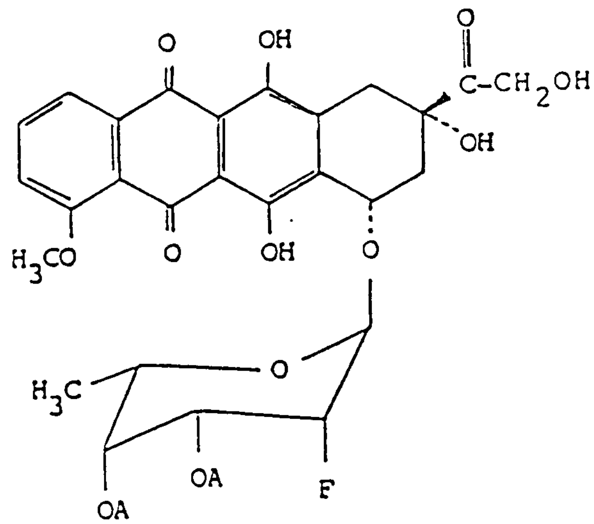

(Ic)

dans laquelle A est comme défini ci-dessus, et à enlever ensuite les groupes protecteurs d'hydroxyle (A) du composé de la formule (Ic) lorsque ce composé de la formule (Ic) contient des groupes protecteurs d'hydroxyle (A) résiduels, pour obtenir le composé de la formule (Id) ci-dessus.

**8.** Procédé selon la revendication 7, dans lequel on fait réagir une daunomycinone de la formule (II) avec le composé de la formule (III) dans un solvant organique aprotique à une température comprise entre -20 ° C et 80 ° C en la présence d'un ou de plusieurs agents de déshydrohalogénation dans des conditions anhydres.

**9.** Procédé selon la revendication 7, dans lequel le stade dans lequel on transforme le groupe méthyle en le groupe hydroxyméthyle en position 14 du produit déprotégé privé des groupes protecteurs d'hydroxyle (A) du composé de la formule (Ia) ou le groupe méthyle en position 14 du composé de la formule (Ia) consiste à bromurer le groupe méthyle en position 14 en un groupe bromométhyle en position 14 en faisant réagir le groupe méthyle en position 14 avec du brome à une température comprise entre 0 ° C et 50 ° C en présence d'un orthoformate d'alcoyle qui protège le groupe carbonyle en position 13 du composé anthracycline, à savoir le produit déprotégé ou le composé de la formule (Ia), et à faire ensuite réagir le groupe bromométhyle en position 14 ainsi formé avec du formate de sodium pour obtenir le groupe hydroxyméthyle en position 14.

**10.** Halogénure de 2,6-didéoxy-2-fluoro-α-L-talopyranosyle de la formule :

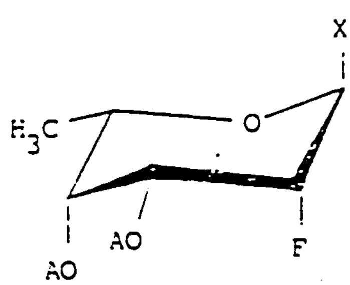

dans laquelle X est un atome de brome, d'iode ou de chlore, et A est un groupe protecteur d'hydroxyle ou un atome d'hydrogène.

**11.** Composé selon la revendication 10, qui est le bromure de 3,4-di-O-acéty-2,6 -didéoxy-2-fluoro-α-L-talopyranosyle.

**12.** Méthyl-2,6-didéoxy-2-fluoro-α-L-talopyranoside.

**13.** 1,3,4-tri-O-acétyl-2,6-didéoxy-2-fluoro-α-L-talopyranose.

**Revendications pour l'Etat contractant suivant : ES**

**1.** Procédé pour la production d'un dérivé d'anthracycline représenté par la formule :

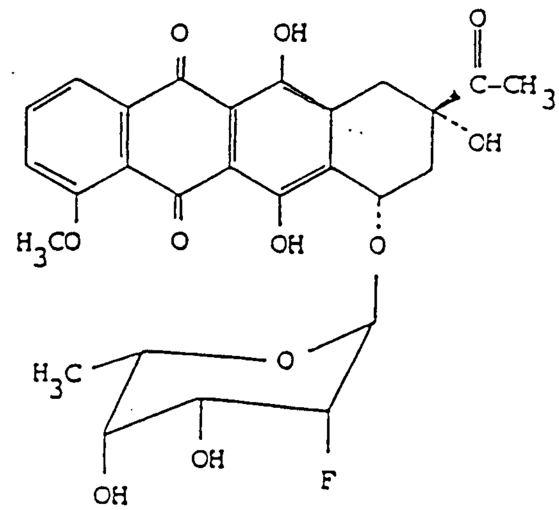

(Ib )

qui consiste à faire réagir une daunomycinone de la formule :

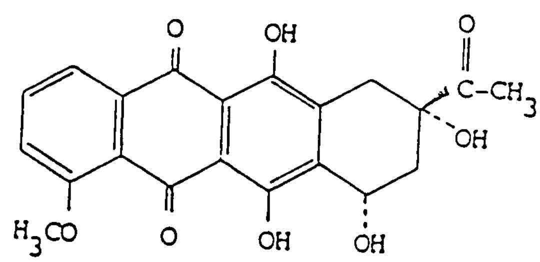

(II)

avec un halogénure de 2,6-didéoxy-2-fluoro-α-L-talopyranosyle de la formule :

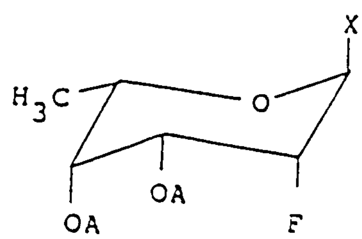

(III)

dans laquelle X est un atome de brome, d'iode ou de chlore et A est un groupe protecteur d'hydroxyle ou un atome d'hydrogène, pour produire un dérivé d'anthracycline de la formule :

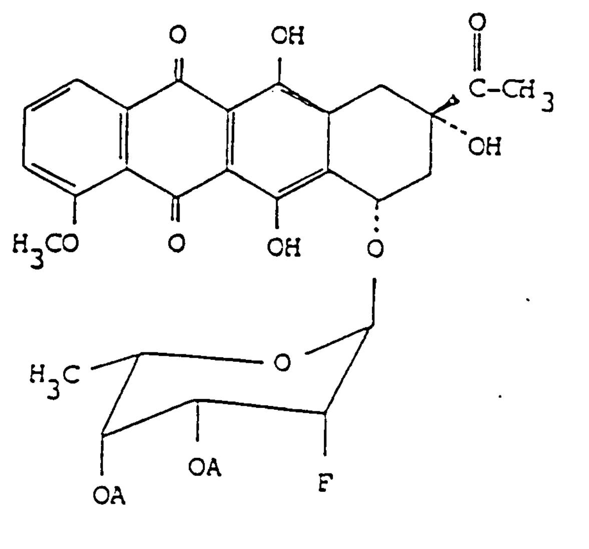

(Ia)

dans laquelle A est comme défini ci-dessus, et à enlever ensuite d'une manière connue les groupes protecteurs d'hydroxyle (A) du composé de la formule (Ia), lorsque le composé de la formule (Ia) contient des groupes protecteurs d'hydroxyle (A) résiduels.

**2.** Procédé selon la revendication 1, dans lequel on fait réagir une daunomycinone de la formule (II) avec le composé de la formule (III) dans un solvant organique aprotique à une température comprise entre -20 °C et 80 °C en la présence d'un ou de plusieurs agents de déshydrohalogénation dans des conditions anhydres.

**3.** Procédé pour la production d'un dérivé d'anthracycline de la formule :

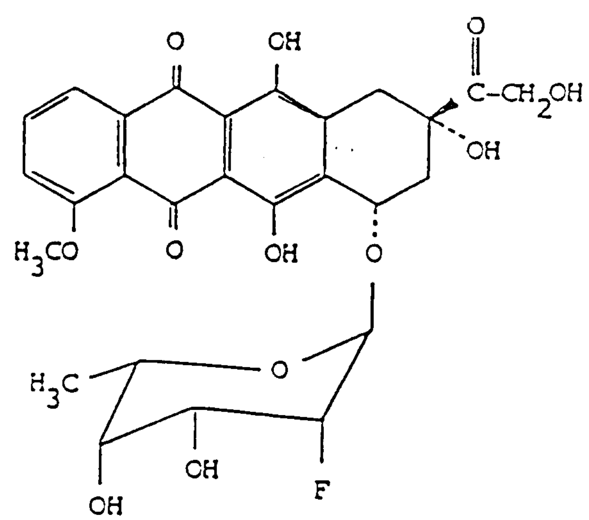

(Id)

qui consiste à faire réagir une daunomycinone de la formule :

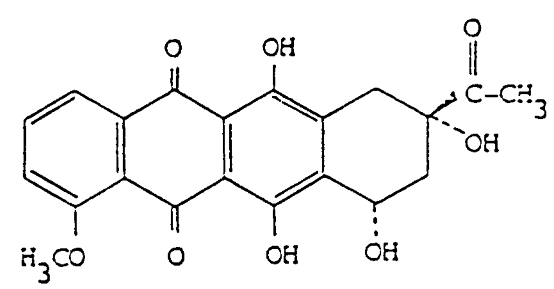

(II)

avec un halogénure de 2,6-didéoxy-2-fluoro-α-L-talopyranosyle de la formule :

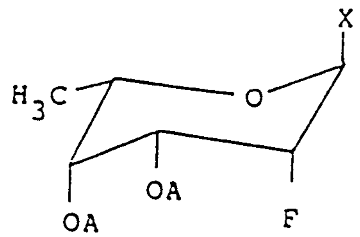

(III)

dans laquelle X est un atome de brome, d'iode ou de chlore, et A est un groupe protecteur d'hydroxyle ou un atome d'hydrogène, pour produire un dérivé d'anthracycline de la formule :

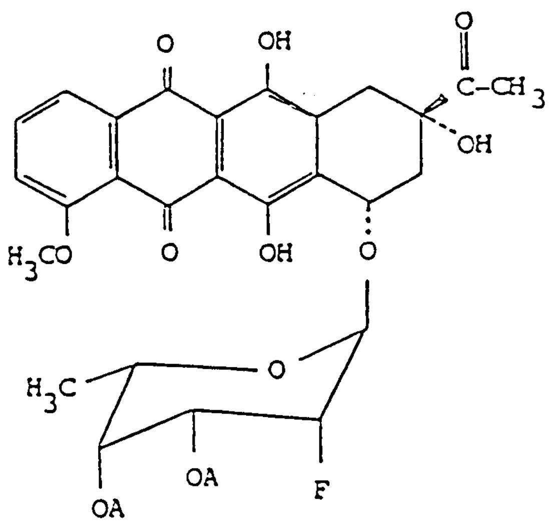

(Ia)

dans laquelle A est comme défini ci-dessus, et ensuite, soit à enlever les groupes protecteurs d'hydroxyle (A) du composé de la formule (Ia) lorsque le composé de la formule (Ia) contient des groupes protecteurs d'hydroxyle (A) résiduels, pour produire le produit déprotégé privé des groupes protecteurs d'hydroxyle (A) et à transformer ensuite le groupe méthyle en position 14 de ce produit déprotégé en un groupe hydroxyméthyle pour produire le composé de la formule (Id) ci-dessus, soit à convertir le groupe méthyle en position 14 du composé de la formule (Ia) en le groupe hydroxyméthyle pour obtenir le dérivé d'anthracycline de la formule :

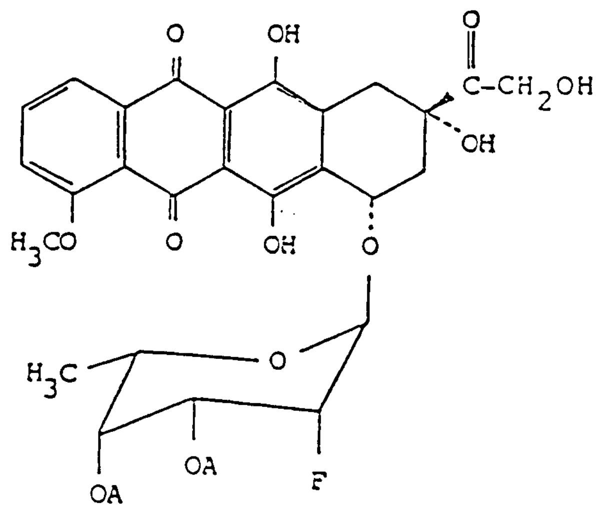

(Ic)

dans laquelle A est comme défini ci-dessus, et à enlever ensuite les groupes protecteurs d'hydroxyle (A) du composé de la formule (Ic) lorsque ce composé de la formule (Ic) contient des groupes protecteurs d'hydroxyle (A) résiduels pour produire le composé de la formule (Id) ci-dessus.

4. Procédé selon la revendication 3, dans lequel on fait réagir une daunomycinone de la formule (II) avec le composé de la formule (III) dans un solvant organique aprotique à une température comprise entre -20 ° C et 80 ° C en la présence d'un ou de plusieurs agents de déshydrohalogénation dans des conditions anhydres.

5. Procédé selon la revendication 3, dans lequel le stade consistant à transformer le groupe méthyle en le groupe hydroxyméthyle dans la position 14 du produit déprotégé privé des groupes protecteurs d'hydroxyle (A) du composé de la formule (Ia) ou le groupe méthyle en la position 14 du composé de la formule (Ia) consiste à bromurer le groupe méthyle en position 14 en le groupe bromométhyle en position 14 en faisant réagir ce groupe méthyle en position 14 avec du brome à une température comprise entre 0 ° C et 50 ° C en présence d'un orthoformate d'alcoyle qui protège le groupe carbonyle en position 13 du composé d'anthracycline, à savoir le produit déprotégé ou le composé de la formule (Ia), et à faire ensuite réagir le groupe bromométhyle en position 14 ainsi formé avec du formate de sodium pour produire le groupe hydroxyméthyle en position 14.

6. Procédé pour la production d'une composition pharmaceutique anti-tumorale contenant un dérivé d'anthracycline représenté par la formule générale :

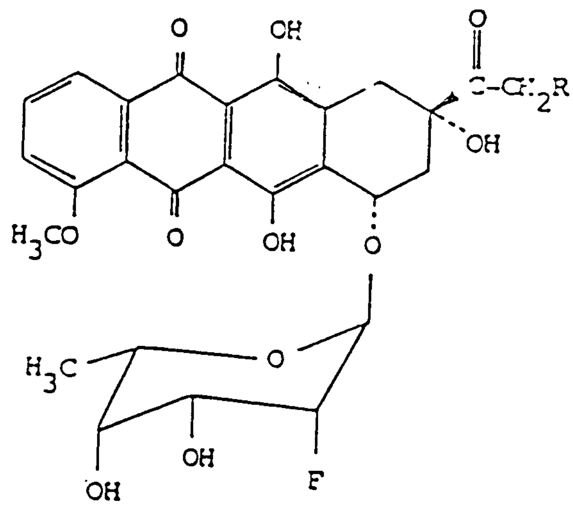

(I)

dans laquelle R est un atome d'hydrogène ou un groupe hydroxyle, en tant qu'ingrédient actif, en

mélangeant ce dérivé d'anthracycline avec un véhicule pharmaceutiquement acceptable.

7. Procédé selon la revendication 6, dans lequel l'ingrédient actif est 7-O-(2,6-didéoxy-2-fluoro-α-L-talopyranosyl)-daunomycinone.

8. Procédé selon la revendication 6, dans lequel l'ingrédient actif est 7-O-(2,6-didéoxy-2-fluoro-α-L-talopyranosyl)-adriamycinone.

9. Procédé pour la préparation d'un halogénure (bromure, iodure ou chlorure) de 2,6-didéoxy-2-fluoro-α-L-talopyranosyle à deux atomes d'oxygène protégés en 3,4, qui consiste à faire réagir le 2,6-didéoxy-2-fluoro-α-L-talopyranose à trois atomes d'oxygène protégés en 1, 3 et 4 avec un tétrahalogénure de titane.

**Patentansprüche**
**Patentansprüche für folgende Vertragsstaaten : CH, DE, FR, GB, IT, LI, NL**

1. Anthracyclinderivat, dargestellt durch die allgemeine Formel

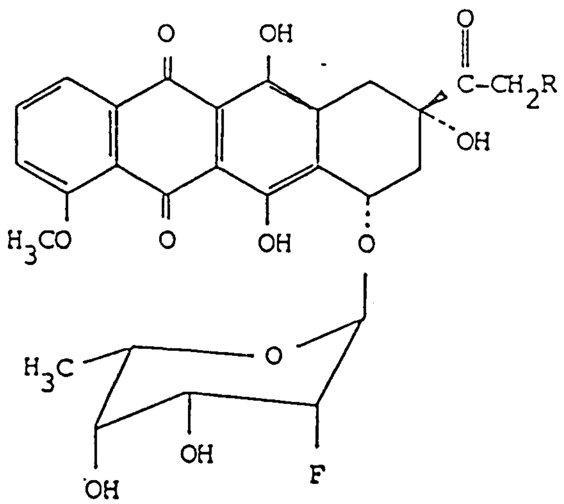

(I)

wobei R ein Wasserstoffatom oder eine Hydroxylgruppe ist.

2. Verbindung nach Anspruch 1, die 7-O-(2,6-Dideoxy-2-fluoro-α-L-talopyranosyl)daunomycinon ist.

3. Verbindung nach Anspruch 1, die 7-O-(2,6-Dideoxy-2-fluoro-α-L-talopyranosyl)adriamycinon ist.

4. Pharmazeutische Antitumorzusammensetzung, die als aktiven Bestandteil ein Anthracyclinderivat, dargestellt durch die allgemeine Formel

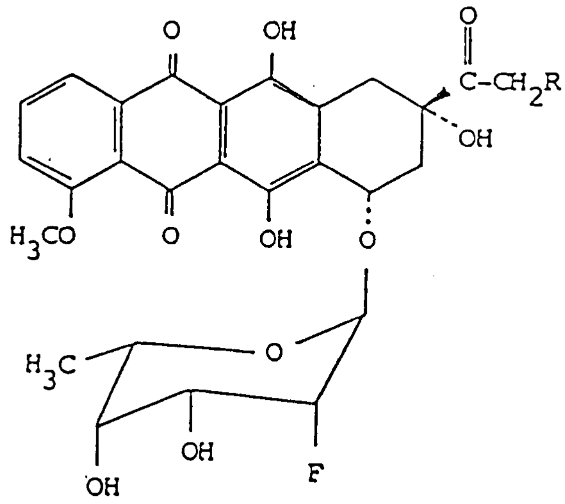

(I)

wobei R ein Wasserstoffatom oder eine Hydroxylgruppe ist, in Verbindung mit einem pharmazeutisch annehmbaren Träger für den aktiven Bestandteil aufweist.

**5.** Verfahren zur Herstellung eines Anthracyclinderivats, dargestellt durch die Formel

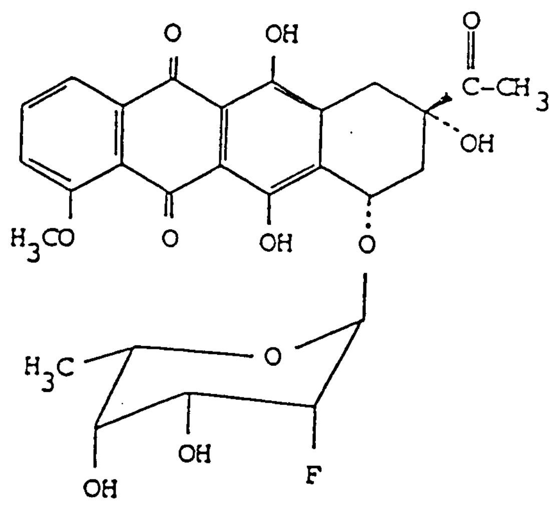

(Ib )

wobei das Verfahren aufweist: Umsetzen von Daunomycinon der Formel

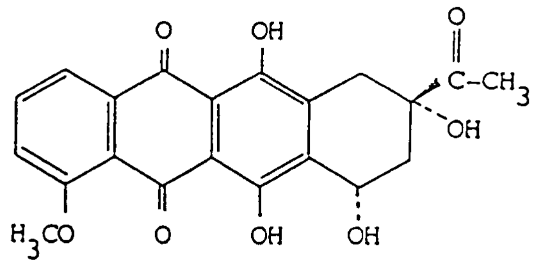

(II)

mit einem 2,6-Dideoxy-2-fluoro-α-L-talopyranosylhalogenid der Formel

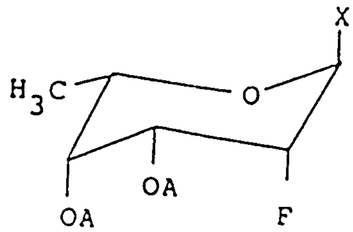

(III)

wobei X ein Brom-, Iod- oder Chloratom und A eine Hydroxylschutzgruppe oder ein Wasserstoffatom ist, unter Erzeugung eines Anthracyclinderivats der Formel

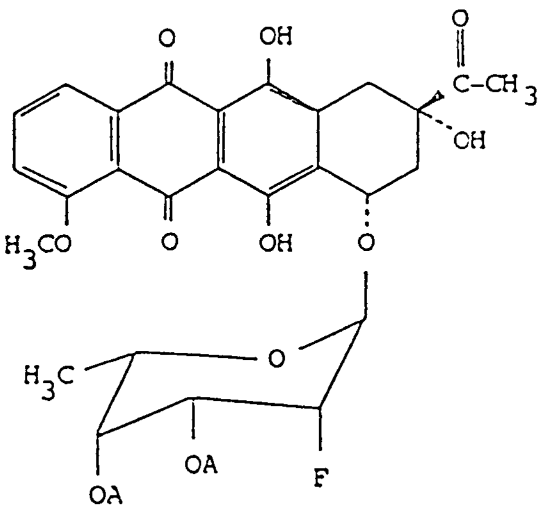

(Ia)

in der A wie oben definiert ist, und anschließendes Entfernen der Hydroxylschutzgruppen (A) aus der Verbindung der Formel (Ia) in bekannter Weise, wenn die Verbindung der Formel (Ia) die darin verbliebenen Hydroxylschutzgruppen (A) enthält.

**6.** Verfahren nach Anspruch 5, wobei Daunomycinon der Formel (II) mit der Verbindung der Formel (III) in einem aprotischen organischen Lösungsmittel bei einer Temperatur von -20 °C bis 80 °C in Anwesenheit von ein oder mehreren dehydrohalogenierenden Mitteln unter wasserfreien Bedingungen umgesetzt wird.

**7.** Verfahren zur Herstellung eines Anthracyclinderivats der Formel

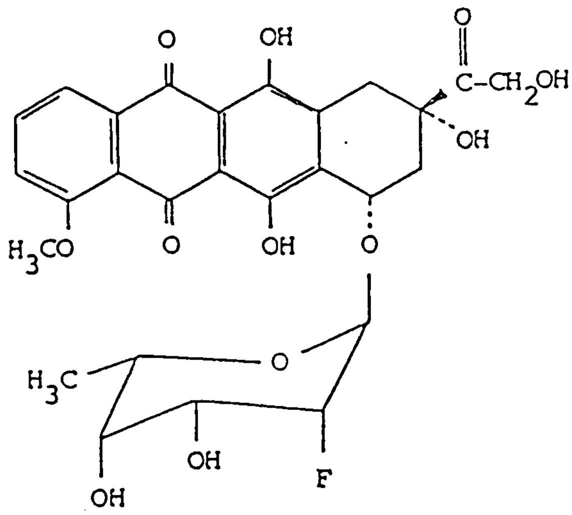

(Id)

wobei das Verfahren aufweist: Umsetzen von Daunomycinon der Formel

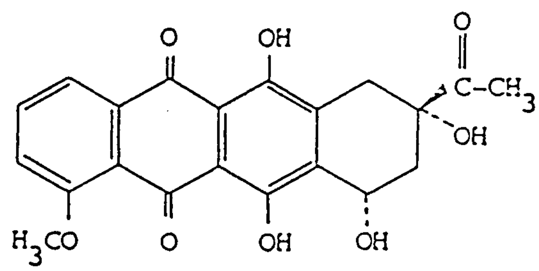

(II)

mit einem 2,6-Dideoxy-2-fluoro-α-L-talopyranosylhalogenid der Formel

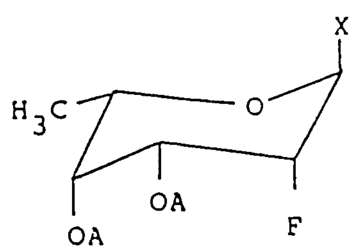

(III)

wobei X ein Brom-, Iod- oder Chloratom und A eine Hydroxylschutzgruppe oder ein Wasserstoffatom ist, unter Erzeugung eines Anthracyclinderivats der Formel

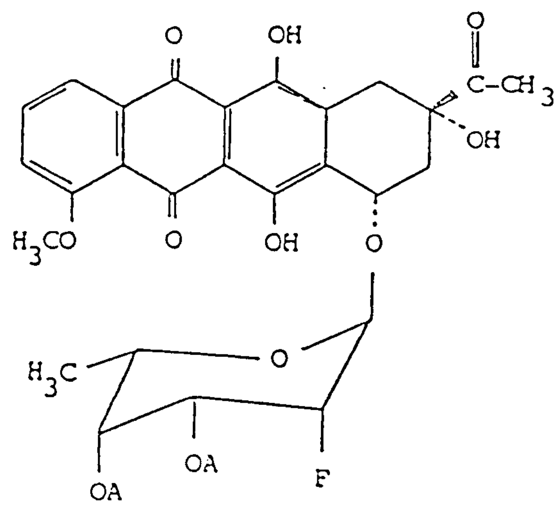

(Ia)

wobei A wie oben definiert ist, und dann entweder Entfernen der Hydroxylschutzgruppen (A) aus der Verbindung der Formel (Ia), wenn die Verbindung der Formel (Ia) die darin verbliebenen Hydroxylschutzgruppen (A) enthält, unter Erzeugung des ungeschützten Produkts, aus dem die Hydroxylschutzgruppen (A) entfernt sind, und anschließendes Umwandeln der Methylgruppe in Stellung 14 des ungeschützten Produkts in eine Hydroxymethylgruppe unter Erzeugung der Verbindung der obigen Formel (Id) oder Umwandeln der Methylgruppe in Stellung 14 der Verbindung der Formel (Ia) in die Hydroxymethylgruppe zum Erhalt eines Anthracyclinderivats der Formel

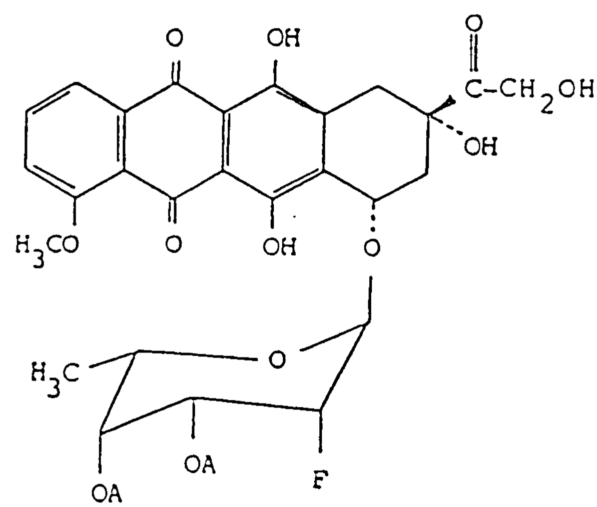

(Ic)

wobei A wie oben definiert ist, und anschließendes Entfernen der Hydroxylschutzgruppen (A) aus der Verbindung der Formel (Ic), wenn diese Verbindung der Formel (Ic) die darin verbliebenen Hydroxylschutzgruppen (A) enthält, unter Bildung der Verbindung der obigen Formel (Id).

**8.** Verfahren nach Anspruch 7, wobei Daunomycinon der Formel (II) mit der Verbindung der Formel (III) in einem aprotischen organischen Lösungsmittel bei einer Temperatur von -20 °C bis 80 °C in Anwesenheit von ein oder mehr dehydrohalogenierenden Mitteln unter wasserfreien Bedingungen umgesetzt wird.

**9.** Verfahren nach Anspruch 7, wobei der Schritt der Umwandlung der Methylgruppe in die Hydroxymethylgruppe in Stellung 14 des ungeschützten Produkts nach Entfernen der Hydroxylschutzgruppen (A) aus der Verbindung der Formel (Ia) oder der Methylgruppe in Stellung 14 der Verbindung der Formel (Ia) aufweist: Bromieren der 14-Methylgruppe zu einer 14-Brommethylgruppe durch Umsetzen der 14-Methylgruppe mit Brom bei einer Temperatur von 0 °C bis 50 °C in Anwesenheit eines Alkylorthoformats, das die 13-Carbonylgruppe der Anthracyclinverbindung, und zwar das ungeschützte Produkt oder die Verbindung der Formel (Ia), schützt, und anschließendes Umsetzen der so gebildeten 14-(Brommethyl)gruppe mit Natriumformat unter Bildung der 14-(Hydroxymethyl)gruppe.

**10.** 2,6-Dideoxy-2-fluoro-α-L-talopyranosylhalogenid der Formel

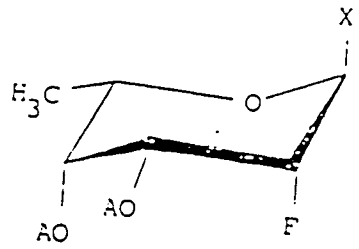

wobei X ein Brom-, Iod- oder Chloratom und A eine Hydroxylschutzgruppe oder ein Wasserstoffatom ist.

**11.** Verbindung nach Anspruch 10, die 3,4-Di-O-acetyl-2,6-dideoxy-2-fluoro-α-L-talopyranosylbromid ist.

**12.** Methyl-2,6-dideoxy-2-fluoro-α-L-talopyranosid.

**13.** 1,3,4-Tri-O-acetyl-2,6-dideoxy-2-fluoro-α-L-talopyranose.

**Patentansprüche für folgenden Vertragsstaat : ES**

**1.** Verfahren zur Herstellung eines Anthracyclinderivats, dargestellt durch die Formel

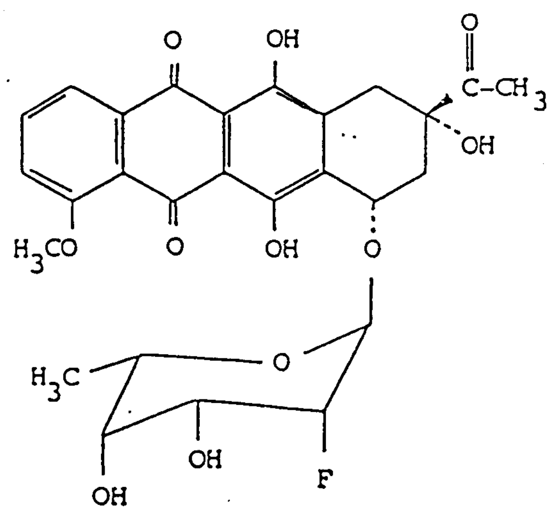

(Ib )

wobei das Verfahren aufweist: Umsetzen von Daunomycinon der Formel

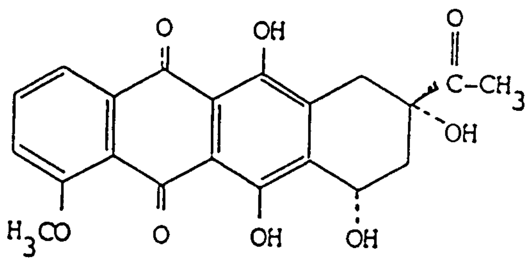

(II)

mit einem 2,6-Dideoxy-2-fluoro-αL-talopyranosylhalogenid der Formel

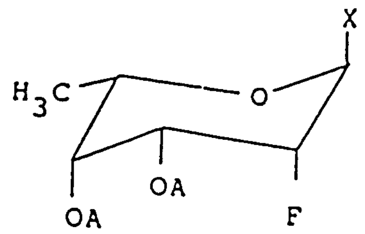

(III)

wobei X ein Brom-, Iod- oder Chloratom und A eine Hydroxylschutzgruppe oder ein Wasserstoffatom ist, unter Erzeugung eines Anthracyclinderivats der Formel

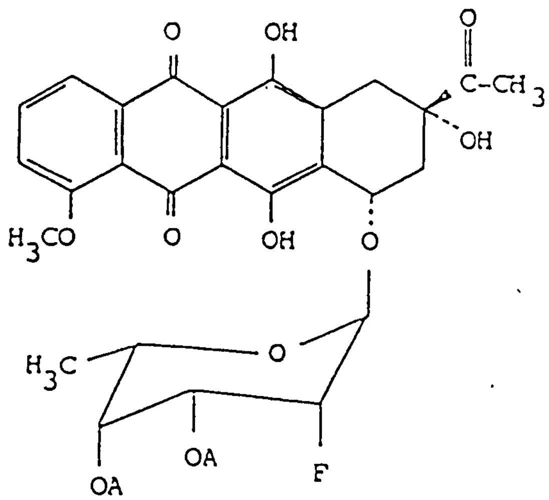

(Ia)

in der A wie oben definiert ist, und anschließendes Entfernen der Hydroxylschutzgruppen (A) aus der Verbindung der Formel (Ia) in bekannter Weise, wenn die Verbindung der Formel (Ia) die darin verbliebenen Hydroxylschutzgruppen (A) enthält.

**2.** Verfahren nach Anspruch 1, wobei Daunomycinon der Formel (II) mit der Verbindung der Formel (III) in einem aprotischen organischen Lösungsmittel bei einer Temperatur von -20 °C bis 80 °C in Anwesenheit von ein oder mehreren dehydrohalogenierenden Mitteln unter wasserfreien Bedingungen umgesetzt wird.

**3.** Verfahren zur Herstellung eines Anthracyclinderivats der Formel

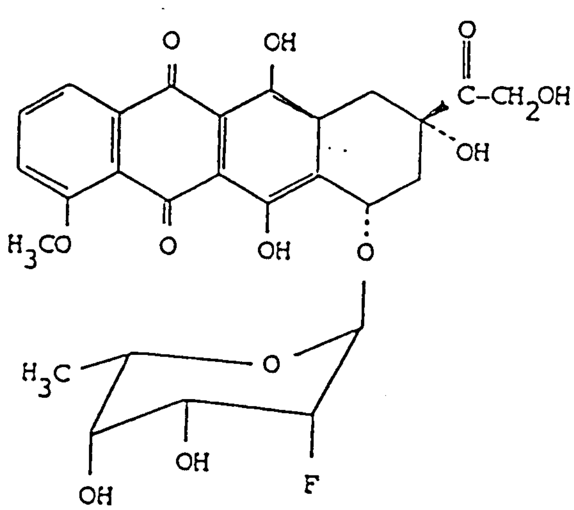

(Id)

wobei das Verfahren aufweist: Umsetzen von Daunomycinon der Formel

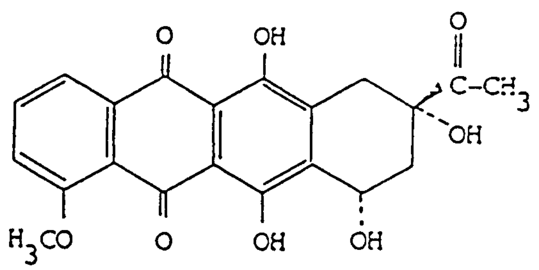

(II)

mit einem 2,6-Dideoxy-2-fluoro-α-L-talopyranosylhalogenid der Formel

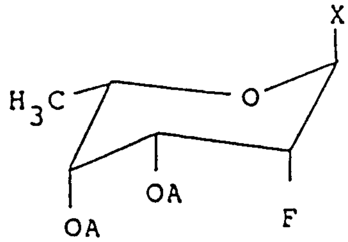

(III)

wobei X ein Brom-, Iod- oder Chloratom und A eine Hydroxylschutzgruppe oder ein Wasserstoffatom ist, unter Erzeugung eines Anthracyclinderivats der Formel

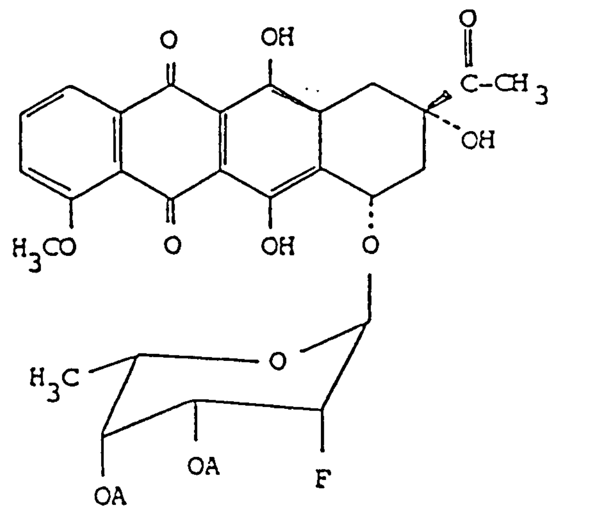

(Ia)

wobei A wie oben definiert ist, und dann entweder Entfernen der Hydroxylschutzgruppen (A) aus der Verbindung der Formel (Ia), wenn die Verbindung der Formel (Ia) die darin verbliebenen Hydroxylschutzgruppen (A) enthält, unter Erzeugung des ungeschützten Produkts, aus dem die Hydroxylschutzgruppen (A) entfernt sind, und anschließendes Umwandeln der Methylgruppe in Stellung 14 des ungeschützten Produkts in eine Hydroxymethylgruppe unter Erzeugung der Verbindung der obigen Formel (Id) oder Umwandeln der Methylgruppe in Stellung 14 der Verbindung der Formel (Ia) in die Hydroxymethylgruppe zum Erhalt eines Anthracyclinderivats der Formel

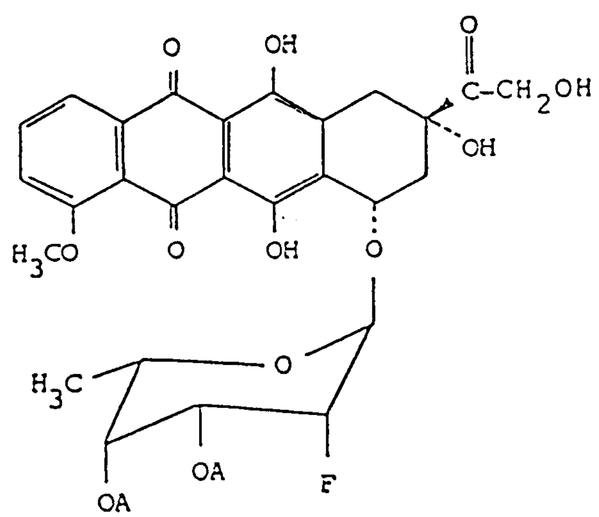

(Ic)

wobei A wie oben definiert ist, und anschließendes Entfernen der Hydroxylschutzgruppen (A) aus der Verbindung der Formel (Ic), wenn diese Verbindung der Formel (Ic) die darin verbliebenen Hydroxylschutzgruppen (A) enthält, unter Bildung der Verbindung der obigen Formel (Id).

4. Verfahren nach Anspruch 3, wobei Daunomycinon der Formel (II) mit der Verbindung der Formel (III) in einem aprotischen organischen Lösungsmittel bei einer Temperatur von -20 °C bis 80 °C in Anwesenheit von ein oder mehr dehydrohalogenierenden Mitteln unter wasserfreien Bedingungen umgesetzt wird.

5. Verfahren nach Anspruch 3, wobei der Schritt der Umwandlung der Methylgruppe in die Hydroxymethylgruppe in Stellung 14 des ungeschützten Produkts nach Entfernen der Hydroxylschutzgruppen (A) aus der Verbindung der Formel (Ia) oder der Methylgruppe in Stellung 14 der Verbindung der Formel (Ia) aufweist: Bromieren der 14-Methylgruppe zu einer 14-Brommethylgruppe durch Umsetzen der 14-Methylgruppe mit Brom bei einer Temperatur von 0 °C bis 50 °C in Anwesenheit eines Alkylorthoformats, das die 13-Carbonylgruppe der Anthracyclinverbindung, und zwar das ungeschützte Produkt oder die Verbindung der Formel (Ia), schützt, und anschließendes Umsetzen der so gebildeten 14-(Brommethyl)gruppe mit Natriumformat unter Bildung der 14-(Hydroxymethyl)gruppe.

6. Verfahren zur Herstellung einer pharmazeutischen Antitumorzusammensetzung, die ein Anthracyclinderivat, dargestellt durch die allgemeine Formel

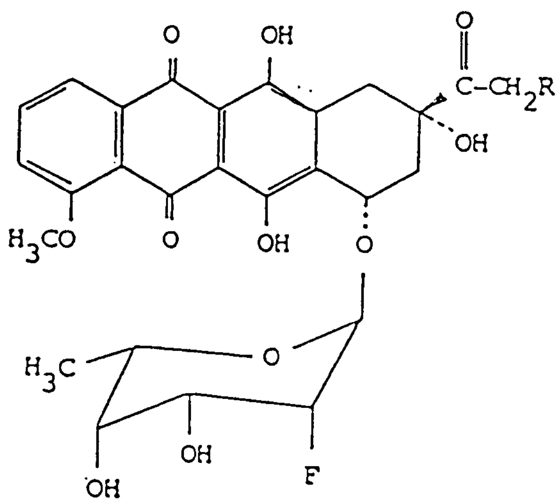

(I)

wobei R ein Wasserstoffatom oder eine Hydroxylgruppe ist, als aktiven Bestandteil aufweist, durch Vermischen des Anthracyclinderivats mit einem pharmazeutisch annehmbaren Träger.

7. Verfahren nach Anspruch 6, wobei der aktive Bestandteil 7-O-(2,6-Dideoxy-2-fluoro-α-L-talopyranosyl)-daunomycinon ist.

8. Verfahren nach Anspruch 6, wobei der aktive Bestandteil 7-O-(2,6-Dideoxy-2-fluoro-α-L-talopyranosyl)-adriamycinon ist.

9. Verfahren zur Herstellung von 3,4-di-O-geschütztem 2,6-Dideoxy-2-fluoro-α-L-talopyranosylhalogenid (Bromid, Iodid oder Chlorid), wobei das Verfahren die Umsetzung von 1,3,4-tri-O-geschützter2,6-Dideoxy-2-fluoro-α-L-talopyranose mit einem Titantetrahalogenid aufweist.